Europäisches Patentamt

European Patent Office

Office européen des brevets

(11) Veröffentlichungsnummer: **0 434 616 B1**

(12) **EUROPÄISCHE PATENTSCHRIFT**

(45) Veröffentlichungstag der Patentschrift: **15.11.95**

(21) Anmeldenummer: **90810970.5**

(22) Anmeldetag: **11.12.90**

(51) Int. Cl.⁶: **C12N 15/87**, C12M 3/00, C12M 1/00, C12N 5/10, A01H 5/00, C12N 1/21

(54) Verfahren und Vorrichtung zur genetischen Transformation von Zellen.

(30) Priorität: **19.12.89 CH 4562/89**

(43) Veröffentlichungstag der Anmeldung:
**26.06.91 Patentblatt 91/26**

(45) Bekanntmachung des Hinweises auf die
Patenterteilung:
**15.11.95 Patentblatt 95/46**

(84) Benannte Vertragsstaaten:
**AT BE CH DE ES FR GB LI**

(56) Entgegenhaltungen:
**EP-A- 0 301 749**
**WO-A-91/00915**

**PROCEEDINGS OF THE NATIONAL ACADEMY OF SCIENCES OF THE USA, Band 86, Nr. 19, Oktober 1989, Seiten 7500-7504, Washington, DC, US; P. CHRISTOU et al.:"Inheritance and expression of foreign genes in transgenic soybean plants"**

**IDEM**

(73) Patentinhaber: **CIBA-GEIGY AG**
**Klybeckstrasse 141**
**CH-4002 Basel (CH)**

Patentinhaber: **SCHWEIZERISCHE EIDGENOS-SENSCHAFT EIDGENÖSSISCHE TECHNISCHE HOCHSCHULE (ETH)**
**ETH-Zentrum**
**Rämistrasse 101**
**CH-8092 Zürich (CH)**

(72) Erfinder: **Sautter, Christof, Dr.**
**Christegässli 3**
**CH-8197 Rafz (CH)**
Erfinder: **Waldner, Heinz**
**Riedgrabenweg 28**
**CH-8050 Zürich (CH)**
Erfinder: **Potrykus, Ingo, Prof. Dr.**
**Im Stigler 54**
**CH-4312 Magden (CH)**

BIOTECHNOLOGY, Band 6, Nr. 5, Mai 1988, Seiten 559-563, New York, US; T.M. KLEIN et al.: "Factors influencing gene delivery into zea mays cells by high-velocity microprojectiles"

TRENDS IN BIOTECHNOLOGY, Band 6, Nr. 12, Dezember 1988, Seiten 299-302,Elsevier Science Publishers Ltd, GB; J.C. SANFORD: "The biolistic process"

NATURE, Band 327, Nr. 6117. 7. - 13. Mai 1987, Seiten 70-73, London, GB; T.M. KLEIN et al.: "High-velocity miroprojectiles for delivering nucleic acids into living cells"

**Beschreibung**

Die Erfindung betrifft ein Verfahren zur genetischen Transformation von Zellen, insbesondere Pflanzenzellen gemäss dem Oberbegriff des Anspruchs 1 sowie eine zur Ausführung dieses Verfahrens geeignete Vorrichtung zum Einbringen von Partikeln in Zellen gemäss dem Oberbegriff des Anspruchs 14. Ein derartiges Verfahren und eine entsprechende Vorrichtung sind zum Beispiel in der EP-A-0 270 356 beschrieben.

Weiterhin betrifft die vorliegende Erfindung die Verwendung des erfindungsgemässen Verfahrens zur Herstellung transgener Pflanzen sowie die mit Hilfe dieses Verfahrens erhältlichen transgenen Pflanzen selbst und deren Nachkommen.

Für die genetische Manipulation des pflanzlichen Erbgutes mit Hilfe der rekombinanten DNA Technologie stehen mittlerweile eine Vielzahl von Verfahren und Techniken zur Verfügung, die in vielen Laboratorien bereits routinemässig angewendet werden.

Zu den am besten untersuchten und am häufigsten verwendeten Verfahren zählt zweifellos das Agrobacterium-Transformationssystem.

Agrobacterium-Zellen besitzen auf ihrem Ti-Plasmid ein grosses DNA-Fragment, die sogenannte T-DNA-Region, das bei der natürlichen Transformation pflanzlicher Zellen in das Pflanzengenom integriert wird.

Dieses natürliche Gen-Transfer-System kann nach Ausführung verschiedener Modifikationen als Gen-Vektor-System für die gezielte Transformation von Pflanzen verwendet werden [Chilton, MD, 1983].

Das Agrobacterium-Transformationssystem hat aber den entscheidenden Nachteil, dass der Wirtsbereich der Agrobakterien auf bestimmte dikotyle Pflanzen sowie auf einige wenige Vertreter der Monokotyledonen (Hernalsteens et al, 1984; Hookas-Van-Slogteren et al., 1984), die aber aus agrarökonomischer Sicht unbedeutend sind, beschränkt ist. Das bedeutet, dass die wichtigsten Kulturpflanzen für einen effektiven Gentransfer nicht zugänglich sind.

Darüberhinaus handelt es sich bei den verwendeten Agrobakterien um Pathogene, die bei ihren Wirtspflanzen charakteristische Krankheitssymptome in Form von krebsartigen Gewebewucherungen auslösen und daher nur unter Wahrung strenger Sicherheitsauflagen im Labor gehandhabt werden dürfen.

Alternative Transformationssysteme, die entwickelt wurden, um diese Nachteile des Agrobacterium Transformationssystems auszugleichen und die auf eine Einschleusung exogener DNA in pflanzliche Protoplasten abzielen, wie der direkte Gentransfer Vektorfreier DNA in Protoplasten (Paszkowski et al., 1984, Potrykus et al., 1986) sowie die Mikroinjektion Vektor-freier DNA in Protoplasten (Steinbiss and Stabel, 1983; Morikawa and Yamada, 1985) oder Zellen (Nomura and Komamine, 1986), müssen insofern als problematisch angesehen werden, als die Regeneration ganzer Pflanzen aus pflanzlichen Protoplasten einer Vielzahl von Pflanzenspezies, insbesondere aus der Gruppe der Gramineen, zur Zeit noch zahlreiche Probleme mit sich bringt.

Ein weiterer Nachteil dieser alternativen Transformations-Systeme betrifft die nach wie vor relativ geringen Transformationsraten, die zur Zeit etwa bei Werten zwischen 1 % und 5 % liegen.

Diese niedrigen Transformationsraten machen es weiterhin notwendig, die einzuschleusende DNA mit Markern (z.B. Antibiotika-Resistenzgene) zu versehen, die eine rasche Selektionierung der Transformanten aus der grossen Zahl nichttransformierter Zellen ermöglichen.

Dies bedeutet aber, dass zur Zeit kein befriedigendes Transformations-Verfahren zur Verfügung steht, das eine auch unter kommerziellen Gesichtspunkten effiziente und kostengünstige Produktion transgener Pflanzen mit neuen und nützlichen Eigenschaften erlaubt, insbesondere was die Pflanzen aus der Gruppe der Monocotyledoneae angeht.

Es muss daher als eine vordringliche Aufgabe angesehen werden, Verfahren zu entwickeln, die eine schnelle, effiziente und reproduzierbare Transformation aller Pflanzen, unabhängig von ihrer taxonomischen Stellung und den damit verbundenen Besonderheiten, erlauben und somit eine auch unter kommerziellen Gesichtspunkten effektive und wirtschaftliche Produktion transgener Pflanzen gewährleisten.

In besonderem Masse trifft dies auf Pflanzen aus der Gruppe der Monocotyledoneae zu, insbesondere auf solche aus der Familie Gramineae, in der die aus agrarökonomischer Sicht bedeutendsten Kulturpflanzen wie Weizen, Gerste, Roggen, Hafer, Mals, Reis, Hirse u.a.m. enthalten sind und die daher von ganz besonderem wirtschaftlichem Interesse sind, insbesondere da für die Herstellung transgener monokotyler Pflanzen bisher noch keine befriedigenden Verfahren zur Verfügung stehen.

Erste Ansätze in dieser Richtung liefern verschiedene, erst in jüngster Zeit entwickelte Transformations-Verfahren, die auf einer Einschleusung von DNA in pflanzliche Zellen beruhen, die innerhalb einer höher organisierten Einheit vorliegen, wie z.B. einem intakten Geweberverband, einem Embryo oder einer ganzen, bereits vollständig entwickelten Pflanze.

Dabei handelt es sich zum einen um die Injektion exogener DNA in die jungen Blütenstände von Roggenpflanzen (de la Pena et al., 1987), zum anderen um eine Agrobacterium-vermittelte Virusinfektion von Mals-Pflanzen mit "Maize Streak Virus" (Grimsley et al., 1987). Doch auch diese neu entwickelten Verfahren sind mit Nachteilen verbunden; so hat sich beispielsweise das zuerst genannte Verfahren als bisher nicht reproduzierbar erwiesen.

Ein alternatives Verfahren, das ebenfalls die Transformation von pflanzlichen Zellen innerhalb einer höher organisierten Einheit zum Ziel hat, basiert auf dem Beschuss dieser Zellen mit Partikeln, die mit der zu transformierenden DNA assoziiert sind. Durch den Aufprall dieser hochbeschleunigten Partikel werden Löcher in die Zellwände der getroffenen Zellen geschlagen, durch die die Partikel einschliesslich der mit diesen assoziierten DNA in die Zelle gelangen.

Mit diesen sogenannten Mikroprojektilen lassen sich sehr schnell eine Vielzahl von Zellen erreichen. Mikroprojektile sind in der Vergangenheit schon zum Gentransfer benutzt worden (Klein et al. 1988; Christou et al., 1988; EP-A-0 270 356) und haben sich für gewisse Fragestellungen bewährt. Zum Beschuss von kleinen Gewebspartien mit entsprechend kleinen Zellen, wie dies z.B. bei Meristemen der Fall ist, eignen sich die käuflichen ballistischen Apparaturen wenig. Da die Metallpartikeln bei ballistischen Verfahren trocken geschossen werden, schiesst man fast stets Aggregate aus mehreren bis vielen Partikeln, die auf der Probe tiefe Wunden reissen. Durch die Bindung der DNA auf den Partikeln wird die Neigung zu Aggregation noch gefördert.

Die generell sehr hohe Partikelgeschwindigkeit der ballistischen Verfahren erfordert einen hohen Arbeitsabstand, der zu einer grossen Streuung der Partikeln führt. Die Partikelgeschwindigkeit kann bei den bekannten ballistischen Verfahren nur in grossen Stufen beeinflusst werden. Die starke Streuung der Partikeln kann daher kaum verbessert werden. Kleine Gewebe mit kleinen Zellen erfordern für hohe Transformationsraten jedoch eine dichte Belegung der Probe mit möglichst einzelnen, gleichmässig verteilten Partikeln auf einem eng begrenzten Zielfeld. Dazu sollte der Impuls der Partikeln möglichst ähnlich sein, d.h. Geschwindigkeit und Masse, die Partikeldichte auf der Probe und die Partikelgeschwindigkeit sollten sich möglichst fein regeln lassen, um an verschiedene Gewebe anpassungsfähig zu bleiben.

Diese Anforderungen werden durch das durch die Merkmale des Anspruchs 1 gekennzeichnete erfindungsgemässe Verfahren bzw. durch die durch die Merkmale des Anspruchs 14 gekennzeichnete erfindungsgemässe Vorrichtung erfüllt. Vorteilhafte Ausgestaltungen und Weiterbildungen sind in den abhängigen Ansprüchen beschrieben.

Die prioritätsältere EP-A-0 405 696 beschreibt eine Vorrichtung zur genetischen Transformation von Zellen, bei der in einer druckfesten Kapillare ein definiertes Volumen einer DNA und Goldpartikel enthaltenden Suspension bereitgestellt und dieses Volumen durch Beaufschlagung mit einem unter Druck stehenden Treibgas an einem Ende der Kapillare als im wesentlichen zusammenhängender Flüssigkeitsfaden oder als turbulentes Flüssigkeits-Gas-Gemisch aus dem anderen Ende der Kapillare bzw. einer daran anschliessenden Zielkanüle herausgeschleudert, beim Austreten zerstäubt und dann auf die zu transformierenden Zellen gerichtet wird.

Gemäss dem erfindungsgemässen Verfahren werden dagegen Mikroprojektile in Form von Partikeln, insbesondere Goldpartikeln, sehr definierter Grösse in Suspension mit einer DNA-Lösung innerhalb einer Druckkammer bereitgestellt und daselbst mit einem Druckstoss in einen feinen Nebel zerstäubt. Die Tröpfchen des Nebels enthalten die Partikeln und die DNA und sind nur wenig grösser als die Partikeln selbst. Der Nebel wird dann mit demselben Druckstoss durch eine feine Pore oder Kapillare aus der Druckkammer herausgepresst gepresst und dabei beschleunigt. Beim Auftreffen auf den Zellen schlagen die Partikeln Löcher in die Zellwände und die Plasmamembran, durch welche die DNA in dem mitgeführten Nebeltröpfchen in die Zellen eintreten kann. Auf einer Zielfläche kleiner als 1 mm Durchmesser kann die Partikeldichte auf der Probe sowie die Geschwindigkeit der Partikeln in weiten Grenzen fein geregelt werden. Die erfindungsgemässe Vorrichtung ist technisch sehr einfach und relativ kostengünstig herzustellen und funktioniert zuverlässig.

Besonders geeignet und damit bevorzugt für die Verwendung in dem erfindungsgemässen Transformationsverfahren sind wenig-zellige Gewebeverbände, insbesondere aber meristematische Gewebeverbände, wie sie z.B. in den Sprossmeristemen von Pflanzen, in Proembryonen und Embryonen oder aber in embryogenen Zellkulturen vorliegen.

Im folgenden wird die Erfindung anhand der Zeichnung näher erläutert. Es zeigen:

Fig. 1     eine Prinzipskizze eines Ausführungsbeispiels der erfindungsgemässen Vorrichtung,

Fig. 2     einen Längsschnitt durch die wesentlichsten Teile der Vorrichtung in grösserem Massstab und

Fig. 3     einen Schnitt nach der Linie III-III der Fig. 2.

Darstellungsgemäss umfasst die erfindungsgemässe Vorrichtung eine langgestreckte, im wesentlichen etwa zylindrische bzw. rohrförmige Druckkammer 1, eine sich daran anschliessende Probenkammer 2, eine Einrichtung 3 zur Erzeugung eines Druckstosses in der Druckkammer, Dosiermittel 4 zur Zufuhr von Suspension in die Druckkammer und eine an die Probenkammer anschliessbare Saugquelle z.B. in Form einer Wasserstrahlvakuumpumpe 5.

Die Einrichtung 3 zur Erzeugung eines Druckstosses umfasst eine an das hintere Ende (nicht dargestellt) angeschlossene $CO_2$-Pistole 31 sowie eine mit dieser in Verbindung stehende $CO_2$-Vorratsflasche 32. Anstelle einer $CO_2$-Pistole könnte auch eine Druckluftpistole und entsprechend anstelle der $CO_2$-Vorratsflasche 32 eine Druckluft-Vorratsflasche vorgesehen sein. Ferner kann die $CO_2$-Pistole auch mit Patronen betreibbar ausgebildet sein, wobei die Vorratsflasche 32 dann entfallen kann. Wesentlich ist lediglich, dass die Einrichtung dazu befähigt ist, in der Druckkammer sehr gleichmässige Druckstösse definierter Grösse im Bereich von etwa einigen bar bis etwa einigen hundert bar oder gegebenenfalls auch höher zu erzeugen. Ausserdem sollen vorzugsweise auch eine Vielzahl von Druckstössen hintereinander mit einer relativ hohen Repetitionsfrequenz (etwa 1 Schuss/s) erzeugbar sein.

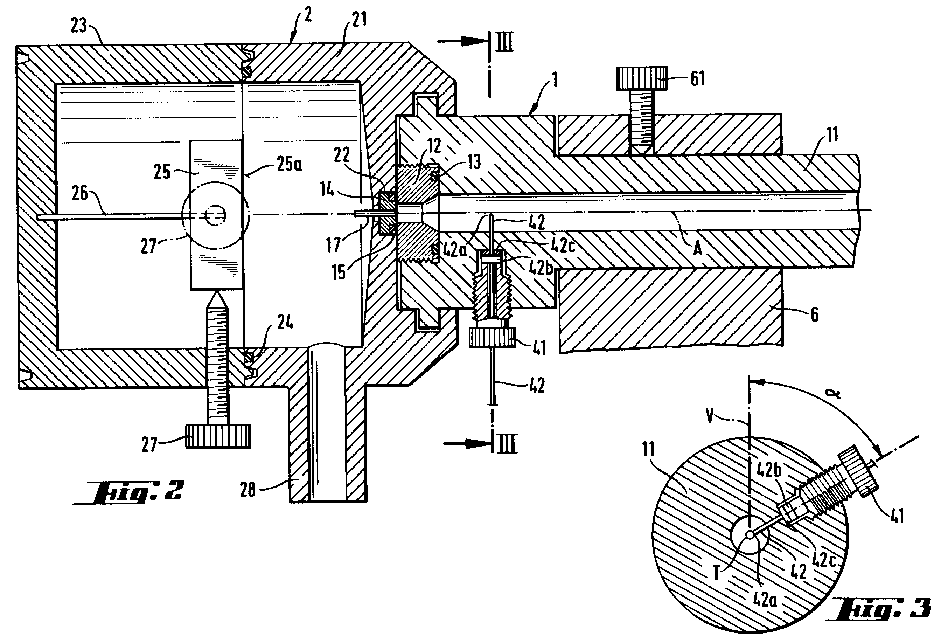

Der Aufbau der Druckkammer 1 ist in Fig. 2 im Detail erkennbar. Sie besteht im wesentlichen aus einem relativ dickwandigen, druckfesten Stahlrohr 11 von etwa 50 mm Länge und etwa 3 mm Innendurchmesser, welches in einem ortsfesten Lagerblock 6 im wesentlichen etwa horizontal eingespannt und um sein Längsachse A drehbar angeordnet ist. Eine Feststellschraube 61 fixiert das Rohr 11 in seiner jeweiligen Position.

Am hinteren, in der Zeichnung rechten Ende des Stahlrohrs 11 ist die Mündung der $CO_2$-Pistole 31 in geeigneter Weise direkt angeschlossen (nicht dargestellt). Im vorderen Ende des Rohrs 11 ist ein Verjüngungsring 12 eingesetzt und mittels eines O-Rings 13 abgedichtet. Der Verjüngungsring reduziert den Innendurchmesser des Stahlrohrs etwa konisch in Richtung auf das Rohrende hin auf etwa die Hälfte.

Auf dem vorderen Ende des Stahlrohrs 11 bzw. der Druckkammer ist ein im wesentlichen becherförmiger Basisteil 21 der als Ganzes im wesentlichen etwa zylindrischen Probenkammer 2 mittels eines Bajonettverschlusses oder dgl. dichtend und lösbar befestigt. Der Basisteil 21 ist mit einer zylindrischen Vertiefung 22 versehen, welche einen Kapillarenhalter 14 aufnimmt und diesen unter Zwischenlage eines weiteren O-Rings 15 dichtend gegen die äussere Stirnfläche des Verjüngungsrings 12 presst. In den Kapillarenhalter 14 ist eine Kapillare 17 dicht und mit der Längsachse A der Druckkammer 1 exakt fluchtend eingesetzt. Die Kapillare 17 verbindet somit den Innenraum der Druckkammer 1 mit dem Innenraum der Probenkammer 2. Anders ausgedrückt, mündet die Druckkammer 1 in eine durch die Kapillare 17 gebildete Restriktion aus. Anstelle der Kapillare 17 kann, wie weiter unten noch näher erläutert, auch eine Restriktion in Form einer Strömungsblende oder allgemein einer Pore vorgesehen sein.

Die Probenkammer 2 besteht aus dem schon genannten, auf dem Stahlrohr 11 lösbar befestigten Basisteil 21 und einem weiteren, ebenfalls im wesentlichen etwa becherförmigen Wandteil 23, welcher lösbar mit dem Basisteil 21 verbunden ist, wobei ein weiterer O-Ring 24 für die nötige Abdichtung der beiden Teile sorgt. Im vom Basisteil 21 abnehmbaren Wandteil 23 befindet sich ein plattenförmiger Zellenträger 25, welcher auf einem elastischen Tragarm 26 aus Federstahl montiert ist. Seine ebene Stirnfläche 25a, die zur Fixierung der mit der Vorrichtung zu beschiessenden Zellen dient, steht im wesentlichen senkrecht auf die Längsachse A der Druckkammer 1. Der Zellenträger 25 kann mittels zweier Stellschrauben 27, die im abnehmbaren Wandteil 23 der Probenkammer 2 angeordnet sind, in zwei zueinander senkrechten Richtungen quer zur Längsachse A der Druckkammer 1 verstellt und damit relativ zur Restriktion bzw. Kapillare 17 justiert werden. Die zweite Stellschraube steht senkrecht zur Zeichenebene. Der mit dem Stahlrohr 11 verbundene Basisteil 21 der Probenkammer 2 ist mit einem Anschlussstutzen 28 für die Wasserstrahlpumpe 5 oder eine andere geeignete Saugquelle bzw. Evakuationseinrichtung versehen. Die Verbindung von Basisteil 21 und abnehmbarem Wandteil 23 ist im wesentlichen spielfrei ausgebildet, so dass sich die Position des Zellenträgers 25 relativ zur Restriktion 17 nicht verändern kann, wenn der Wandteil 23 abgenommen und wieder aufgesetzt wird.

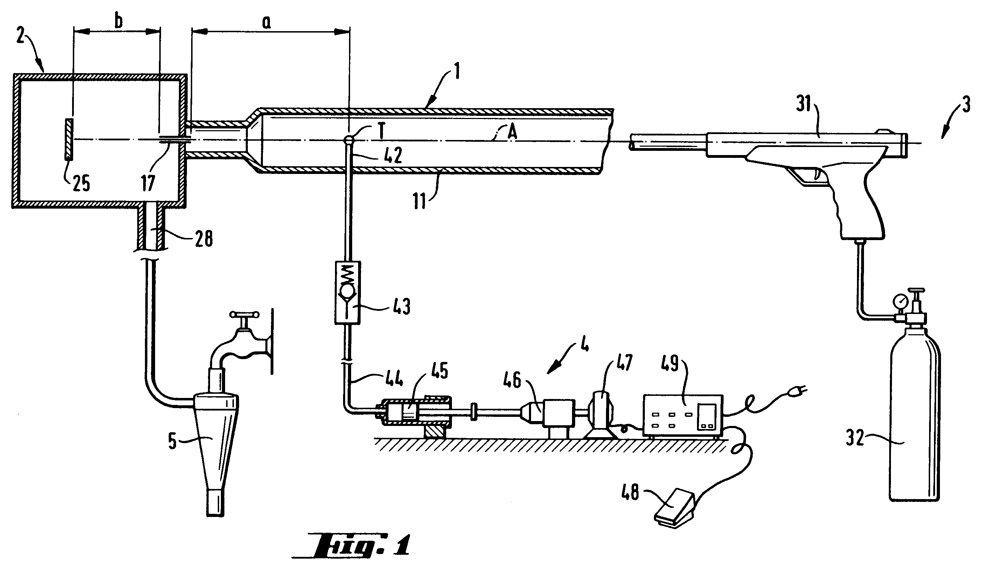

Im vorderen Teil der Druckkammer 1 ist in den Mantel des Stahlrohrs 1 ein Hohlnippel 41 eingeschraubt, durch den eine Stahlkanüle 42 radial in das Innere des Stahlrohrs 11 führt. Zur Fixierung und Abdichtung der Stahlkanüle (Aussendurchmesser etwa 0,75 mm, Innendurchmesser etwa 0,5 mm) sind ein an der Kanüle angeformter Metallwulst 42b und ein Dichtungsring 42c aus PTFE ("Teflon") vorgesehen. Die Mündung 42a der Stahlkanüle 42 liegt knapp vor der Längsachse A der Druckkammer 1, so dass ein aus der Kanüle 42 austretender Flüssigkeitstropfen T (Fig. 1) exakt in der Längsachse A liegt.

Aus zeichnerischen Gründen sind der Hohlnippel 41 und die Stahlkanüle 42 in Fig. 2 vertikal von unten in das Stahlrohr 11 einmündend dargestellt. Im Betrieb der Vorrichtung mündet die Kanüle 42 jedoch schräg von oben unter einem Winkel $\alpha$ zur Vertikalen V in die Druckkammer 1 ein, so wie dies in der Fig. 3 dargestellt ist. Der Winkel $\alpha$ kann durch Verdrehen des Stahlrohrs 11 im Lagerblock 6 den Erfordernissen entsprechend justiert werden (Fixierung durch die Feststellschraube 61).

5

Die Stahlkanüle 42 ist über ein Rückschlagventil 43 und eine flexible Leitung 44 an ein Dosieraggregat angeschlossen, welches aus einer Dosierspritze 45, einem Spindelgetriebe (Mikrometerschraube) 46, einem Schrittmotor 47 und einer über einen Fussschalter 48 in Gang setzbaren elektrischen Steuerung 49 besteht. Das an sich konventionelle und deshalb keiner näheren Erläuterung bedürfende Dosieraggregat ist befähigt, in reproduzierbarer Weise innerhalb weiter, einstellbarer Grenzen Flüssigkeitsmengen (Suspension) im Mikroliterbereich durch die Kanüle 42 in die Druckkammer 1 einzuspeisen, wobei die Flüssigkeit (Suspension) an der Kanülen-Mündung 42a tropfenweise austritt.

Die generelle Funktions- bzw. Betriebsweise der Vorrichtung ist wie folgt:

Die zu beschiessenden Zellen werden bei abgenommenem Wandteil 23 der Probenkammer 2 in weiter unten noch näher erläuterter Weise auf der Oberfläche 25a des Zellenträgers 25 befestigt. Danach wird der Wandteil 23 auf den Basisteil 21 aufgesetzt und die Probenkammer 2 mittels der Wasserstrahlpumpe 5 evakuiert. Hierauf wird die mit einer DNA-Lösung und darin suspendierten Mikroprojektilen in Form von feinsten Goldpartikeln geladene Dosiereinrichtung 4 in Gang gesetzt und so an der Mündung 42a der Kanüle 42 ein Suspensions-Tropfen T geeigneter Grösse (siehe folgende Detailerläuterung) bereitgestellt. Daraufhin wird die $CO_2$-Pistole 31 aktiviert und damit in der Druckkammer 1 ein sich im wesentlichen in Längsrichtung A der Kammer fortpflanzender Druckstoss definierter Grösse erzeugt. Dieser Druckstoss zerstäubt den an der Kanülenmündung 42a bereitgehaltenen Suspensions-Tropfen T in einen feinen Nebel, der die Goldpartikel und daran anhaftende DNA enthält. Die Goldpartikel reissen somit die DNA aus der flüssigen Phase heraus und führen sie auf diese Weise mit sich. Dieser feine Nebel wird nun durch den Druckstoss durch die durch die Kapillare 17 gebildete Restriktion gepresst und dabei erheblich beschleunigt. Die aus der Kapillare 17 austretenden Nebeltröpfchen, welche die Goldpartikel mit der daran haftenden DNA enthalten, treffen nun innerhalb eines sehr kleinen Streukegels mit grosser Geschwindigkeit und definiertem Impuls auf die auf dem Zellenträger 25 fixierten Zellen auf und dringen in diese ein. Bei geeigneter Dimensionierung der Vorrichtung und der Verfahrensparameter kann dabei erreicht werden, dass jeweils nur ein Partikel oder höchstens einige wenige Partikel in die Zellen eindringen und in diesen auch tatsächlich verbleiben. Ferner ist durch die Vorrichtung eine extrem hohe Zielgenauigkeit (geringe Streuwirkung) gewährleistet.

Die Probenkammer 2 ist (teil-)evakuiert. Dadurch wird die Reibung der sich von der Restriktion 17 auf den Zellenträger 25 hin bewegenden Nebeltröpfchen herabgesetzt, die Tröpfchengrösse minimiert und die Richtungsstreuung klein gehalten. Ausserdem spannt das (Teil-)Vakuum die Zellwände, so dass sie sich von den auftreffenden Partikeln besser durchschlagen lassen. Uebliches Wasserstrahlpumpen-Vakuum (etwa 500 mmHg) ist im Normalfall ausreichend, für spezielle Anwendungen (z.B. im Falle von widerstandsfähigeren Zellwänden) ist auch höheres Vakuum (geringerer Druck) möglich.

Wie schon erwähnt, endet die Kanüle 42 knapp vor der Längsachse A der Druckkammer 1, so dass der an der Kanülen-Mündung 42a erzeugte Suspensions-Tropfen genau in der Längsachse A zu liegen kommt. Auf diese Weise wird eine zur Längsachse symmetrische Tröpfchenwolke erzielt, die wiederum die Zielgenauigkeit und die Verteilung der Partikeln im Auftreffbereich günstig beeinflusst.

Der Abstand a (Fig. 1) zwischen der Mündung 42a der Kanüle 42 und der Eintrittsöffnung der Kapillare 17 bzw. der Blende oder Pore ist relativ kritisch. Ist dieser Abstand zu gering, dann wird nicht der Nebel, d.h. einzelne Nebeltröpfchen, durch die Restriktion gepresst, sondern es tritt ein geschlossener Flüssigkeitsfaden als Strahl aus der Restriktion aus in die Probenkarnmer. Ein solcher Strahl führt zu massiven Zerstörungen der zu beschiessenden Zellen und muss daher unter allen Umständen vermieden werden. In der Praxis muss daher der Abstand a mindestens etwa 5-10 mm betragen, wobei dieser Mindestwert auch von den Dimensionen der übrigen Teile der Vorrichtung abhängt und jeweils empirisch ermittelt werden muss.

Der Arbeitsabstand b (Fig. 1) zwischen dem Austrittsende der Kapillare 17 bzw. Restriktion allgemein und dem Zellenträger 25, d.h. diejenige Strecke, welche die Nebeltröpfchen im freien Flug im Vakuum zurücklegen, ist weniger kritisch. Praktische Werte sind 5 mm bis 5 cm, abhängig vom Gewebetyp und von der Art der Restriktion. Der Arbeitsabstand beeinflusst über die (durch das Teilvakuum reduzierte) Reibung die Partikelgeschwindigkeit und über die Streuung den effektiven Zielflächendurchmesser. Ein Wert von ca. 10 mm hat sich als günstig erwiesen.

Wie schon erwähnt, kann als Restriktion sowohl eine Kapillare als auch eine Strömungsblende oder allgemein Pore eingesetzt werden. Als Blende kommt z.B. eine solche aus Platin-Iridium in Frage, wie sie in der Elektronenmikroskopie verwendet wird. Der Oeffnungsdurchmesser der Blenden kann beispielsweise von 20 $\mu$m bis 500 $\mu$m oder sogar bis zu 1 mm betragen. Bevorzugt werden Durchmesser im Bereich von etwa 70-300 $\mu$m. Im Falle von Kapillaren können diese aus Glas oder Metall (z.B. Stahl) bestehen. Für den Innendurchmesser gelten dieselben Werte wie für die Blenden. Die Länge der Kapillaren kann etwa 0,1-20 mm betragen, bevorzugt werden Werte im Bereich von etwa 1,0-10 mm. Das Verhältnis von Innendurchmesser zu Länge der Kapillare bestimmt den Strömungswiderstand. Längere Kapillaren erfordern daher in der Regel grössere Innendurchmesser. Je länger die Kapillare ist, desto grösser ist die Beschleunigungsstrecke für die Nebeltröpfchen und umso grösser ist ihre Endgeschwindigkeit beim Eintritt in die Probenkammer.

Der Winkel $\alpha$, unter dem die Kanüle 42 zur Vertikalen V geneigt in die Druckkammer 1 einmündet (Fig. 3), ist durch Verdrehen des Rohrs 1 im Lagerblock 6 einstellbar. Da die etwa 1,2 $\mu$m bis 1,5 $\mu$m grossen, in der DNA-Lösung suspendierten Goldpartikeln ohne Rühren sedimentieren (absinken), ist der Winkel $\alpha$ vorteilhafterweise so einzustellen, dass bei jeder Volumendosierung im wesentlichen gleich viele Goldpartikeln in den Tropfen an der Mündung der Kanüle 42 austreten. Bei ungünstigem Winkel $\alpha$ sind sonst die Goldpartikeln vorwiegend in den ersten Tropfen ($\alpha$ zu klein) oder in den letzten Tropfen ($\alpha$ zu gross) einer Serie konzentriert. Der optimale Winkel hängt von der Partikelgrösse und der Viskosität der Lösung ab und muss durch Ausprobieren ermittelt werden.

Ein wesentliches Element der Vorrichtung ist das in der Kanüle 42 (oder ev. einer Zuleitung zu dieser) befindliche Rückschlagventil 43. Es besteht aus einem Ventilsitz aus Quarz und einer Ventilkugel aus Saphir und ist vorzugsweise vertikal orientiert. Das Rückschlagventil 43 ist wie die übrigen Teile der Dosiereinrichtung 4 mit flüssigem Paraffin (Marke 7174 der Firma MERCK, Darmstadt, BRD) luftblasenfrei gefüllt.

Das Dosieraggregat 45-49 kann an sich beliebig realisiert sein, sofern es im Stande ist über einen Zeitraum von maximal etwa 20 Sekunden ein Volumen von etwa 1 $\mu$l bis 10 $\mu$l zu pumpen. In einer praktischen Ausführung wird ein Volumen von 1 $\mu$l bis maximal 4 $\mu$l in Pumpschritten von etwa 1 nl bis etwa 50 nl dosiert. Vorzugsweise ist ein 3-Weg-Ventil vorgesehen, über das eine 1 ml-Spritze zuschaltbar ist, damit das Paraffin bequem nachgefüllt und das Volumen justiert werden kann.

Die die Goldpartikeln enthaltende Suspension wird in die Kanüle 42 eingefüllt. (Zu diesem Zweck wird die Kanüle vorübergehend aus der Druckkammer 1 entfernt.) Die Förderung der Suspension erfolgt indirekt durch Verdrängung durch das dosiert gepumpte flüssige Paraffin. Das Füllvolumen der Kanüle 42 mit Suspension beträgt beispielsweise etwa 3-4 $\mu$l.

Die Zerstäubungswirkung, d.h. die Grösse der Nebeltröpfchen, hängt von der Grösse des Druckstosses in der Druckkammer 1 ab. Anderseits bestimmt die Grösse des Druckstosses auch die Gasgeschwindigkeit in der Restriktion und damit die Partikelgeschwindigkeit bzw. deren Impuls. Der Druckstoss kann zwischen etwa einigen bar und einigen tausend bar liegen, praktische Werte sind einige zehn bar bis einige hundert bar, insbesondere ein Wert von ungefähr 50 bar.

Die Partikelgrösse hat über die Masse einen linearen Einfluss auf den Impuls der Partikeln. Für das Ueberleben der getroffenen Zellen sind prinzipiell kleine Partikeln günstiger. Anderseits können grössere Partikeln besser in die Zellen eindringen. Die optimale Partikelgrösse muss daher für jeden Zelltyp experimentell ermittelt werden. Praktische Partikelgrössen liegen im Bereich von 20 nm bis 5 $\mu$m, insbesondere im Bereich von etwa 1-2 $\mu$m, speziell etwa 1,2-1,5 $\mu$m.

Das Partikelmaterial ist an sich sekundär. Günstig ist jedoch eine hohe spezifische Dichte und grosse Trägheit bei chemischen Reaktionen (inertes Material). Günstig ist weiterhin, wenn die Partikeln untereinander in einem engen Grössenbereich liegen, also alle Partikeln im wesentlichen etwa gleich gross sind. Für die Praxis ist es weiterhin günstig, wenn Partikeln verwendet werden, die bei guter Reproduzierbarkeit eines engen Grössenspielraums in einem weiten Grössenbereich einfach herstellbar sind. Vorzugsweise wird daher als Material für die Partikeln Gold verwendet. Goldpartikeln einheitlicher Grösse lassen sich aus Tetrachlorgoldsäure durch Reduktion mit photographischen Entwicklern gut reproduzierbar herstellen. Durch Verdünnung der Ausgangslösungen lassen sich Partikeln zwischen 5 $\mu$m Durchmesser bis hinab in den kolloidalen Bereich (kleiner als 0,5 $\mu$m Durchmesser) herstellen. Kritisch ist das Abstoppen des Entwicklungsvorgangs mit einem Fixativ für gut reproduzierbare Goldpartikelgrössen sowie für leicht wasserlösliche Reaktionsprodukte, die durch Zentrifugation und Waschen mit Wasser entfernt werden müssen. Die fertigen Goldsuspensionen werden autoclaviert.

Eine relativ kritische Grösse ist die Partikeldichte in der Suspension. Stockende Suspensionen kann man nicht handhaben. Stark verdünnte Suspensionen geben zu viele leere Tröpfchen und zu wenige Treffer in den Zellen. Dazwischen liegt ein Optimum, das für die entsprechenden Zelltypen ermittelt werden muss. Ziel ist es, möglichst viele Zellen mit nur einem einzigen Partikel zu treffen, da weitere Partikeln die Ueberlebenschancen der getroffenen Zellen drastisch reduzieren. Ein praktischer Wert liegt bei $10^{10}$ Partikeln/ml.

Das Suspensionsvolumen, das pro "Schuss" möglich ist, d.h. das Volumen des bereitgestellten Tropfens sollte so gross wie möglich sein. Ist das vorgelegte Suspensionsvolumen pro Schuss jedoch zu hoch, so kommt es zu einer Flüssigkeitsansammlung in der Restriktion und zum Austritt eines geschlossenen Flüssigkeitsfadens, der die Zellen stark verletzt. Ein praktischer Wert bei Restriktionsinnendurchmessern um 100 $\mu$m ist z.B. etwa 0,1 $\mu$l, bei Durchmessern um 300 $\mu$m ist ein Tropfenvolumen von etwa 0,2 $\mu$l

günstig. Ein Wert von 0,5 $\mu$l hat sich in diesem Fall als zu hoch erwiesen. Im allgemeinen können Tropfenvolumina zwischen 10 nl und 500 nl verwendet werden. Besonders bevorzugt ist ein Tröpfchenvolumen zwischen 50 nl und 150 nl.

Die DNA-Konzentration in der Suspension kann in weiten Grenzen zwischen Sättigung bis hinunter zu extremen Verdünnungen gewählt werden. Für die Plasmide pBl221 und pHP 23 sind beispielsweise Werte um 0,3 $\mu$g/$\mu$l geeignet. Die Plasmide können dabei entweder in linearisierter oder überspiralisierter ("supercoiled") Form vorliegen. Grundsätzlich kann von einer höheren DNA-Konzentration eine höhere Transformationsrate erwartet werden.

Wird eine Restriktion in Form einer Blende verwendet, so hat die Grösse der Nebeltröpfchen für ein Suspensionstropfenvolumen von 0,2 $\mu$l bei ca. 70 $\mu$m Innendurchmesser der Blende ein Minimum. Kleine Tröpfchen sind essentiell wegen der Reibung beim Flug und wegen der Bremswirkung auf die Partikeln beim Aufschlag sowie wegen der Verletzungsgefahr am Aufschlagpunkt. Letzteres gilt natürlich auch für Restriktionen in Form einer Kapillare. Die Grösse der Nebeltröpfchen hängt auch vom Volumen des Suspensionstropfens ab und kann daher auch dadurch beeinflusst werden.

Ueblicherweise ist die in die Zellen einzubringende DNA frei in der flüssigen Phase der Suspension gelöst. In gewissen Fällen kann es jedoch auch von Vorteil sein, die Montage der Plasmide auf den Goldpartikeln z.B. mit Hilfe von Spermidin oder Lactoferin zu modifizieren.

Die Grösse der Zielfläche, d.h. derjenigen Fläche, in der mehr als 80 % der Partikel auftreffen, hängt von der Grösse und der Art der Restriktion (Innendurchmesser, Kapillarenlänge etc.), vom Arbeitsabstand b und vom Vakuum ab. Mit einer Kapillare von 300 $\mu$m Innendurchmesser und 10 mm Länge sowie einem Arbeitsabstand von 10 mm und dem genannten Wasserstrahlpumpenvakuum kann mit der vorstehend beschriebenen Vorrichtung ein extrem kleiner Zielflächendurchmesser von maximal 0,7 mm erreicht werden. Der effektive Bereich, in dem Zellen getroffen werden und die Goldpartikeln in die Zellen eindringen, ist sogar noch kleiner. Er hängt auch von der Widerstandfähigkeit (Kohäsion) der Zellwände ab.

Die Partikeldichte auf der Zielfläche ist eine Funktion aus Arbeitsabstand, Vakuum (Streuung), Suspensionsdichte der Vorlage, Restriktionsinnendurchmesser und Kapillarenlänge. Die Partikeldichte auf der Zielfläche sollte so eingestellt werden, das möglichst viele Zellen von genau 1 Partikel getroffen werden. Die übrigen Parameter müssen darauf abgestimmt werden.

Da beim Beschiessen der Zellen ein Gasstrom aus der Restriktion austritt, der die Zellen trifft, ist eine sichere Montage derselben auf dem Zellenträger essentiell. Beispielsweise kann dazu als Unterlage 2 % Agarose auf Thermanox (LAB-TEK Division, Miles Laboratories, Naperville IL 60540, USA) verwendet werden. Die Agarose wird 1 mm dick ausgegossen und enthält 10 mM CaCl$_2$. Die Zellen werden auf der Agarose mit 4 % Alginat festgeklebt. Das Alginat härtet in Gegenwart von Ca$^{2+}$ bei Zimmertemperatur. Nach ca. 1 Stunde ist die Polymerisation abgeschlossen. So montierte Zellen werden vom Gasstrom nicht weggeweht. Diese Montageart lässt sich leicht steril durchführen und geht rasch in der Handhabung und sichert eine ausreichende Wasserversorgung der Zellen bis zu ihrer Rücktransplantation auf das Medium.

Das erfindungsgemässe Verfahren wird unter sterilen Bedingungen durchgeführt. Goldpartikeln, DNA-Lösung und alle Lösungen bzw. Vorrichtungteile, mit denen die Zellen in Berührung kommen, werden steril gehalten durch Autoklavieren, Sterilfiltration oder Abwischen bzw. Durchspülen mit 70 % Aethanol. Die ganze Vorrichtung steht unter einer Sterilbank. Druckkammer und Blende bzw. Kapillare werden ebenfalls von Zeit zu Zeit (bei häufiger Benützung 1 bis 2 mal täglich) mit 70 % Aethanol gespült. Die Suspensionskanüle wird darüberhinaus gelegentlich autoklaviert. Weitere Vorkehrungen zur Sterilität sind im Prinzip nicht erforderlich.

Wie schon erwähnt, kann der Wandteil 23 der Probenkammer 2 mitsamt dem Zellenträger 25 und der Stellschrauben 27 abgenommen werden. Auf diese Weise ist die Justierung der zu beschiessenden Zellen relativ zur Reduktion bzw. zur Längsachse A (Hauptschiessrichtung) sehr einfach. Ein Probeschuss in 2 % Agar markiert die Zielfläche. Anschliessend wird der Wandteil 23 der Probenkammer abgenommen und definiert orientiert und positioniert auf einen Kreuztisch unter einem Stereomikroskop montiert. Jetzt wird der Kreuztisch so justiert, dass der Zielfleck zentral im Fadenkreuz des Okkulars liegt. Die Zellen können jetzt mit den 2 Stellschrauben auf das Fadenkreuz bzw. damit auf die Zielfläche ausgerichtet werden. Selbstverständlich kann die Justierung auch in anderer Weise erfolgen.

Die Vorteile des erfindungsgemässen Verfahrens und der erfindungsgemässen Vorrichtung sind evident: Es wird eine gleichmässige Verteilung einzelner Partikeln auf einer extrem kleinen Zielfläche erreicht. Die Partikeldichte auf der Zielfläche bzw. den beschossenen Zellen, der Impuls der Partikeln und der Durchmesser der Zielfläche sind in weiten Grenzen fein regelbar. DNA muss - obwohl möglich - weder an die Partikeln gekoppelt noch auf die Partikeln präzipitiert werden. Zudem ist die Vorrichtung sehr einfach in der Handhabung und konstruktiv wenig aufwendig.

Das zuvor beschriebene Verfahren zur Herstellung transgener Pflanzen ist mit allen seinen Teilschritten Bestandteil der vorliegenden Erfindung.

Das erfindungsgemässe Verfahren ist universell auf Zellen aller Pflanzen anwendbar, unabhängig von deren taxonomischer Stellung. Besonders geeignet und damit bevorzugt im Rahmen dieser Erfindung ist die Verwendung von Zellen in Geweben, insbesondere in wenig-zelligen Gewebeverbänden, vorzugsweise aber meristematischen Gewebeverbänden, wie sie z.B. in den Sprossmeristemen der Pflanzen, in Proembryonen und Embryonen oder aber in embryogenen Zellkulturen vorliegen. Selbstverständlich kann aber auch jedes beliebige andere geeignete pflanzliche Material in dem erfindungsgemässen Verfahren verwendet werden, wie z.B. Einzelzellen, Zygoten, Zellaggregate, pflanzliche Organe, Gewebe, Kallus, etc..

Das erfindungsgemässe Verfahren ist ebenfalls anwendbar auf andere eukaryontische Zellen wie beispielsweise tierische Zellen oder Zellen von Pilzen, die entweder isoliert oder als Bestandteil einer höher organisierten Einheit vorliegen können, desweiteren Zellen von Algen oder von einzelligen Protozoen, etc.

Das erfindungsgemässe Verfahren ist ebenfalls anwendbar auf prokaryontische Zellen wie bakterielle Zellen und Zellen von Cyanobakterien. Bevorzugt sind bakterielle Zellen ausgewählt aus der Gruppe bestehend aus enterobakteriellen Zellen, insbesondere *E. coli*- und Serratia-Zellen, Agrobakterium-Zellen, Bacillus- Zellen, insbesondere *Bacillus thuringiensis*-und *Bacillus cereus*-Zellen, Streptomyceten-Zellen, Pseudomonaden-Zellen, etc.

Als transformierende DNA können sowohl natürliche DNA-Sequenzen als artifiziell mit Hilfe der rekombinanten DNA-Technologie erzeugte hybride Genkonstruktionen verwendet werden.

In erster Linie umfasst vom breiten Umfang der vorliegenden Erfindung sind rekombinante DNA-Moleküle, die DNA-Sequenzen enthalten, welche bei den pflanzlichen Transformanten zu nützlichen und wünschenswerten Eigenschaften führen.

Dabei handelt es sich vorzugsweise um rekombinante DNA-Moleküle, die eine oder mehrere Gensequenzen beinhalten, die eine nützliche und wünschenswerte Eigenschaft kodieren und die unter der regulatorischen Kontrolle von in Pflanzen aktiven Expressionssignalen stehen, sodass eine Expression besagter Gensequenzen in der transformierten pflanzlichen Zelle gewährleistet ist.

Für die Verwendung in dem erfindungsgemässen Verfahren sind somit in erster Linie alle diejenigen Gene geeignet, die in der pflanzlichen Zelle exprimiert werden und die der Pflanze eine nützliche und/oder gewünschte Eigenschaft verleihen, wie z.B. eine erhöhte Resistenz gegenüber Pathogenen (beispielsweise gegenüber phytophathogenen Pilzen, Bakterien, Viren etc.), eine Resistenz gegenüber Chemikalien [beispielsweise gegenüber Herbiziden (wie z.B. Triazinen, Sulfonylharnstoffen, Imidazolinonen, Triazolpyrimidinen, Bialaphos, Glyphosate, etc.), Insektiziden oder anderen Bioziden]; Resistenz gegenüber nachteiligen (endaphischen oder atmosphärischen) Umwelteinflüssen (beispielsweise gegenüber Hitze, Kälte, Wind, besondere, extreme Bodenbeschaffenheit, Feuchtigkeit, Trockenheit, osmotischer Stress etc.); oder aber zu einer erhöhten oder aber qualitativ besseren Bildung von Reserve- oder Speicherstoffen in Blättern, Samen, Knollen, Wurzel, Stengeln, etc. führen. Zu den wünschenswerten Substanzen, die von transgenen Pflanzen produziert werden können, zählen beispielsweise Proteine, Stärken, Zucker, Aminosäuren, Alkaloide, Riechstoffe, Farben, Fette, etc.

Ebenso können in dem erfindungsgemässen Verfahren Gene eingesetzt werden, die pharmazeutisch akzeptable Wirksubstanzen, wie z.B. Alkaloide, Steroide, Hormone, Immunmodulatoren, und andere physiologisch aktive Substanzen kodieren.

Eine Resistenz gegenüber Cytotoxinen beispielsweise kann durch Übertragung eines Gens erreicht werden, das ein Enzym kodiert und in der pflanzlichen Zelle exprimiert, welches das Cytotoxin detoxifiziert, wie z.B. die Neomycinphosphotransferase Typ II oder die Aminoglycosidphosphotransferase Typ IV, die zu einer Detoxifikation von Kanamycin, Hygromycin und anderen Aminoglykosidantibiotika beitragen oder eine Glutathion-S-Transferase, Cytochrom P-450 oder andere katabolisch wirksame Enzyme, von denen bekannt ist, dass sie Triazine, Sulfonylharnstoffe oder andere Herbizide detoxifizieren. Eine Resistenz gegenüber Cytotoxinen kann auch durch ein Gen vermittelt werden, das in einer Pflanze eine bestimmte Form eines 'Zielenzyms' (Angriffspunkt der Cytotoxinwirkung) exprimiert, die resistent ist gegen die Aktivität des Cytotoxins, wie z.B. eine Variante der Acetohydroxysäuresynthase, die gegenüber der inhibitorischen Wirkung von Sulfonylharnstoffen, Imidazolinonen oder anderen Herbiziden, die mit diesem spezifischen Stoffwechselschritt interagieren, insensitiv ist; oder eine Variante der EPSP Synthase, die sich gegenüber der Hemmwirkung von Glyphosate als insensitv erweist. Es kann sich als vorteilhaft erweisen, diese veränderten Zielenzyme in einer Form zu exprimieren, die ihren Transport in das korrekte zelluläre Kompartiment, wie z.B. im obigen Fall in die Chloroplasten, erlaubt.

In bestimmten Fällen kann es darüberhinaus von Vorteil sein, die Genprodukte in die Mitochondrien, die Vakuolen, das endoplasmatische Retikulum oder in andere Zellregionen, möglicherweise sogar in die interzellulären Räume (Apoplasten) zu dirigieren.

Eine Resitenz gegenüber bestimmten Pilzklassen kann beispielsweise durch die Einschleusung eines Gens erreicht werden, das Chitinase in den pflanzlichen Geweben exprimiert. Zahlreiche pflanzenpathogene Pilze enthalten Chitin als integralen Bestandteil ihrer Hyphen- und Sporenstrukturen, so z.B. die Basidiomyceten (Brand- und Rostpilze), Ascomyceten und *Fungi Imperfecti* (einschliesslich *Alternaria* und *Bipolaris*, *Exerophilum turcicum*, *Colletotricum*, *Gleocercospora* und *Cercospora*). Chitinase ist in der Lage das Mycelwachstum bestimmter Pathogene *in−vitro* zu hemmen. Ein pflanzliches Blatt oder eine Wurzel, die Chitinase konstitutiv oder aber als Antwort auf das Eindringen eines Pathogens exprimiert, ist gegen den Angriff einer grossen Zahl verschiedener Pilze geschützt. Je nach Situation kann eine konstitutive Expression vorteilhaft sein im Vergleich zu einer induzierbaren Expression, die bei zahlreichen Pflanzen als normale Reaktion auf einen Pathogenbefall auftritt, da die Chitinase unmittelbar in hoher Konzentration vorliegt, ohne dass erst eine lag-Phase für die Neusynthese abgewartet werden muss.

Eine Resistenz gegenüber Insekten kann beispielsweise durch ein Gen übertragen werden, das ein Polypeptid kodiert, welches toxisch ist für Insekten und/oder deren Larven, wie z.B. das kristalline Protein von *Bacillus thuringiensis*. Eine zweite Klasse von Proteinen, die eine Insektenresistenz vermitteln, sind die Proteaseinhibitoren. Proteaseinhibitoren bilden einen gewöhnlichen Bestandteil von pflanzlichen Speicherstrukturen. Es konnte nachgewiesen werden, dass ein aus Sojabohnen isolierter und gereinigter Bowman-Birk Proteaseinhibitor die Darmprotease von *Tenebrio* Larven hemmmt [Birk *et al* (1963)]. Das Gen, welches den Trypsininhibitor aus der Kuherbse kodiert, ist bei Hilder et al (1987) beschrieben.

Für die Verwendung in dem erfindungsgemässen Verfahren sind somit in erster Linie all diejenigen Gene geeignet, die bei den transformierten pflanzlichen Zellen sowie den daraus sich entwickelnden Geweben und insbesondere aber den Pflanzen zu einem Schutzeffekt führen, wie z.B. zu einer erhöhten Resistenz gegenüber Pathogenen (beispielsweise gegenüber phytopathogenen Pilzen, Bakterien, Viren, etc.); einer Resistenz gegenüber Chemikalien [beispielsweise gegenüber Herbiziden (wie z.B. Triazinen, Sulfonylharnstoffen, Imidazolinonen, Triazolpyrimidinen, Bialaphos, Glyphosate, etc,), Insektiziden oder anderen Bioziden]; einer Resistenz gegenüber nachteiligen (endaphischen oder atmosphärischen) Umwelteinflüssen (beispielsweise gebenüber Hitze, Kälte, Wind, ungünstigen Bodenverhältnissen, Feuchtigkeit, Trockenheit, etc.).

Gene, die im Rahmen der vorliegenden Erfindung Verwendung finden können umfassen beispielsweise auch solche, die zu einer erhöhten Bildung von Reserve- oder Speicherstoffen in Blättern, Samen, Knollen, Wurzel, Stengeln, etc. führen. Zu den wünschenswerten Substanzen, die von transgenen Pflanzen produziert werden können, zählen beispielsweise Proteine, Stärken, Zucker, Aminosäuren, Vitamine, Alkaloide, Flavine, Riech- und Farbstoffe, Fette, etc..

Ebenso können Strukturgene mit der erfindungsgemässen, regulatorischen DNA-Sequenz assoziiert werden, die pharmazeutisch akzeptable Wirksubstanzen, wie z.B. bestimmte Alkaloide, Steroide, Hormone, Immunmodulatoren und andere physiologisch akitve Substanzen kodieren.

Zu den Genen, die im Rahmen dieser Erfindung in Betracht gezogen werden können, gehören daher bekannte Gene, ohne jedoch darauf beschränkt zu sein, beispielsweise pflanzenspezifische Gene wie das Zeingen aus Mals, das Aveningen aus Hafer, das Glutelingen aus Reis, etc., Säuger-spezifische Gene wie das Insulingen, das Somatostatingen, die Interleucingene, das t-PA Gen, etc., oder aber Gene mikrobiellen Ursprungs wie das NPT II Gen, etc. sowie synthetische Gene wie das Insulingen, etc.

Neben natürlicherweise vorkommenden Strukturgenen, die eine nützliche und wünschenswerte Eigenschaft kodieren, können im Rahmen dieser Erfindung auch Gene verwendet werden, die zuvor mit Hilfe chemischer oder gentechnologischer Methoden gezielt modifiziert wurden.

Weiterhin sind von dem breiten Konzept der vorliegenden Erfindung auch solche Gene umfasst, die vollständig durch chemische Synthese hergestellt werden. Als Gene oder DNA Sequenzen die im Rahmen der vorliegenden Erfindung Verwendung finden können, kommen somit sowohl homologe als auch heterologe Gen(e) oder DNA sowie synthetische Gen(e) oder DNA in Betracht. Als Beispiel für ein synthetisches Gen sein an dieser Stelle das Insulingen genannt.

Weiterhin kann im Rahmen der vorliegenden Erfindung auch sog. 'anti-sense' DNA verwendet werden, die in operabler Verknüpfung mit in pflanzlichen Zellen aktiven Expressionssignalen zur Produktion eines RNA Moleküls führt, das zumindest einem. Teil einer von einer 'sense' DNA kodierten mRNA komplementär ist und diese daher zu binden vermag. Auf diese Weise kann die Translation einer bestimmten mRNA in das korrespondierende Protein verhindert oder aber zumindest eingeschränkt werden, sodass man hiermit ein Instrumentarium in Händen hat mit dessen Hilfe eine effektive Kontrolle der Genexpression von ausgesuchten Genen in der Pflanze möglich wird.

Die im Rahmen der vorliegenden Erfindung verwendbaren DNA Sequenz können ausschliesslich aus genomischer, aus cDNA bzw. synthetischer DNA konstruiert sein. Eine andere Möglichkeit besteht in der Konstruktion einer hybriden DNA-Sequenz bestehend sowohl aus cDNA als auch aus genomischer DNA und/oder synthetischer DNA.

In diesem Fall kann die cDNA aus demselben Gen stammen wie die genomische DNA oder aber, sowohl die cDNA als auch die genomische DNA können aus verschiedenen Genen stammen. In jedem Fall können aber sowohl die genomische DNA und/oder die cDNA, jede für sich, aus dem gleichen oder aus verschiedenen Genen hergestellt sein.

Wenn die DNA-Sequenz Anteile von mehr als einem Gen beinhaltet, können diese Gene entweder von ein und demselben Organismus, von mehreren Organismen, die verschiedenen Stämmen oder Varietäten derselben Art oder verschiedenen Spezies derselben Gattung angehören, oder aber von Organismen, die mehr als einer Gattung derselben oder einer anderen taxonomischen Einheit (Reich) angehören, abstammen.

Um die Expression besagter Strukturgene in der pflanzlichen Zelle zu gewährleisten, ist es vorteilhaft, wenn die kodierenden Gensequenzen zunächst in operabler Weise mit in pflanzlichen Zellen funktionsfähigen Expressions-Sequenzen verknüpft werden.

Jeder Promotor und jeder Terminator, der in der Lage ist, eine Induktion der Expression einer kodierenden DNA-Sequenz (Strukturgen) zu bewirken, kann als Bestandteil der hybriden Gensequenz verwendet werden. Besonders geeignet sind Expressionssignale, die aus Genen von Pflanzen oder Pflanzenviren stammen. Beispiele geeigneter Promotoren und Terminatoren sind z.B. solche der Cauliflower Mosaik Virus Gene (CaMV) oder aber homologe DNA-Sequenzen, die noch die charakteristischen Eigenschaften der genannten Expressionssignale aufweisen. Ebenfalls geeignet sind bakterielle Expressionssignale, insbesondere aber die Expressionssignale der Nopalin-Synthase-Gene (nos) oder der Octopin-Synthase-Gene (ocs) aus den Ti-Plasmiden von *Agrobacierium tumefaciens*.

Bevorzugt im Rahmen dieser Erfindung sind die 35S und 19S Expressions-Signale des CaMV Genoms oder deren Homologe, die mit Hilfe molekularbiologischer Methoden, wie sie z.B. bei Maniatis *et al*,(1982) beschrieben sind, aus besagtem Genom isoliert und mit der kodierenden DNA-Sequenz verknüpft werden können.

Unter Homologen der 35S und 19S Expressionssignale sind im Rahmen dieser Erfindung Sequenzen zu verstehen, die trotz geringer Sequenzunterschiede den Ausgangssequenzen im wesentlichen homolog sind und die nach wie vor die gleiche Funktion wie diese erfüllen.

Als Ausgangsmaterial für die 35S-Transkriptionskontroll-Sequenzen kann erfindungsgemäss z.B. das Scal-Fragment des CaMV Stammes "S", das die Nukleotide 6808-7632 der Genkarte umfasst [Frank G *et al* (1980)], verwendet werden.

Die 19S Promotor- und 5' nicht-translatierte Region befindet sich auf einem Genomfragment zwischen der PstI Stelle (Position 5386) und der HindIII-Stelle (Position 5850) der CaMV Genkarte [Hohn *et al* (1982)]. Die entsprechende Terminator- und 3' nicht-translatierte Region liegt auf einem EcoRV/BglII-Fragment zwischen Position 7342 und 7643 des CaMV Genoms.

Weiterhin bevorzugt im Rahmen dieser Erfindung sind die Expressionssignale des CaMV Stammes CM 1841, dessen komplette Nukleotidsequenz bei Gardner RC *et al* (1981) beschrieben ist.

Ein weiterer wirksamer Vertreter eines Pflanzenpromotors, der Verwendung finden kann, ist ein überproduzierender Pflanzenpromotor. Diese Art von Promotoren sollte, sofern sie in operabler Weise mit der Gensequenz, welche ein gewünschtes Genprodukt kodiert, verknüpft ist, in der Lage sein, die Expression besagter Gensequenz zu vermitteln.

Ueberproduzierende Pflanzenpromotoren, die im Rahmen der vorliegenden Erfindung zur Anwendung kommen können, schliessen den Promotor der kleinen Untereinheit (small subunit; ss) der Ribulose-1,5-bisphosphat-Carboxylase aus Sojabohnen sowie den Promotor des Chlorophyl-a/b- Bindungsproteins ein. Diese beiden Promotoren sind dafür bekannt, dass sie in eukaryontischen Pflanzenzellen durch Licht induziert werden [siehe z.B. Genetic Engineering of Plants, an Agricultural Perspective, Cashmore A (1983)].

Weitere regulatorische DNA-Sequenzen, die für die Konstruktion chimärer Gene Verwendung finden können umfassen beispielsweise solche Sequenzen, die in der Lage sind die Transkription einer assoziierten DNA-Sequenz in pflanzlichen Geweben im Sinne einer Induktion oder Repression zu regulieren.

So gibt es beispielsweise einzelne Pflanzengene, von denen bekannt ist, dass sie durch verschiedene interne und externe Faktoren wie Pflanzenhormone, Hitzeschock, Chemikalien, Pathogene, Sauerstoffmangel, Licht, etc. induziert werden.

Als ein Beispiel für eine Genregulation durch ein Pflanzenhormon soll hier die Abszissinsäure (ABS) erwähnt werden, von der bekannt ist, dass sie den während der späten Embryonalphase auftretenden Überschuss an mRNAs in Baumwolle induziert. Ein weiteres Beispiel liefert die Gibberellinsäure (GA3), die in Rizinussamen Malatsynthasetranskripte sowie in den Aleuronschichten von Gerste Isoenzyme der $\alpha$-Amylase induziert.

Die Aktivität von Glucanase und Chitinase in Bohnenblättern kann durch Behandlung mit dem Stresshormon Ethylen markant erhöht werden. Für Chitinase wird dieser Induktions-Effekt über den Promotor des Chitinasegens gesteuert, was durch Reportergenversuche unter Verwendung eines Promotors aus dem Chitinasegen von Bohnen (*Phaseolus vulgaris*) gezeigt werden konnte.

Die Regulation von Hitzeschock empfindlichen Proteingenen der Sojabohne wurde im Detail untersucht. Eine mehrstündige Behandlung der Pflanzen bei einer Temperatur von 40°C resultiert in der *de novo* Synthese von sogenannten Hitzeschock-Proteinen. Zahlreiche dieser Gene wurden inzwischen isoliert und ihre Regulation im einzelnen analysiert. Die Expression dieser Gene wird in erster Linie auf der Ebene der Transkription kontrolliert. Fusioniert man beispielsweise den Promotor des hps70 Gens mit dem Neomycinphosphotransferase II (NPT II) Gen, so kann das auf diese Weise entstandene chimäre Gen durch einen Hitzeschock induziert werden (Spena et al, 1985).

Eine andere Klasse von in Pflanzen induzierbaren Genen beinhaltet die Licht-regulierten Gene, insbesondere das nukleär kodierte Gen der kleinen Untereinheit der Ribulose-1,5-bisphosphatcarboxylase (RUBISCO). Morelli *et al* (1985) haben nachgewiesen, dass die 5'-flankierende Sequenz eines RUBISCO-Gens aus der Erbse in der Lage ist, die Licht-induzierbarkeit auf ein Reportergen zu übertragen, sofern dies in chimärer Form mit dieser Sequenz verknüpft ist. Diese Beobachtung konnte auch auf andere Licht-induzierte Gene ausgedeht werden, wie z.B. das Chlorophyll a/b -Bindungsprotein.

Die Alkoholdehydrogenase-Gene (adh-Gene) des Mais waren Gegenstand intensiver Untersuchungen. Das adh1-s Gen aus Mais wurde isoliert und es wurde nachgewiesen, dass ein Teil der 5'-flankierenden DNA in der Lage ist, die Expression eines chimären Reportergens (z.B. Chloramphenicolacetyltransferase; CAT) zu induzieren, wenn das vorübergehend transformierte Gewebe anaeroben Bedingungen ausgesetzt wurde [Howard *et al* (1987)].

Eine weitere Gruppe regulierbarer DNA-Sequenzen betrifft chemisch regulierbare Sequenzen, die z.B. in den PR-("pathogenesis related proteine")Proteingenen des Tabaks vorliegen und mit Hilfe chemischer Regulatoren induzierbar sind.

Die zuvor beispielhaft genannten regulierbaren DNA-Sequenzen können sowohl natürlichen als auch synthetischen Ursprungs sein oder aber aus einem Gemisch von natürlichen und synthetischen DNA-Sequenzen bestehen.

Die verschiedenen Sequenzabschnitte können mit Hilfe an sich bekannter Methoden miteinander zu einer vollständigen kodierenden DNA-Sequenz verknüpft werden. Geeignete Methoden schliessen beispielsweise die *in vivo* Rekombination von DNA-Sequenzen, die homologe Abschnitte aufweisen sowie die *in vitro* Verknüpfung von Restriktionsfragmenten mit ein.

Hybride Genkonstruktionen lassen sich durch Einspleissen in einen geeigneten Klonierungsvektor und anschliessende Transformation einer geeigneten Wirtszelle sehr schnell und einfach amplifizieren.

Als Klonierungsvektoren verwendet man im allgemeinen Plasmid- oder Virus-(Bakteriophage)-Vektoren mit Replikations- und Kontrollsequenzen, die von Spezies stammen, die mit der Wirtszelle kompatibel sind.

Der Klonierungsvektor trägt in der Regel einen Replikationsursprung, ausserdem spezifische Gene, die zu phaenotypischen Selektionsmerkmalen in der transformierten Wirtszelle führen, insbesondere zu Resistenzen gegenüber Antibiotika oder gegenüber bestimmten Herbiziden. Die transformierten Vektoren können anhand dieser phaenotypischen Marker nach einer Transformation in einer Wirtszelle selektiert werden.

Selektierbare phaenotypische Marker, die im Rahmen dieser Erfindung Anwendung finden können, umfassen beispielsweise Resistenzen gegen Ampicillin, Tetracyclin, Hygromycin, Kanamycin, Methotrexat, G418 und Neomycin, ohne das diese beispielhafte Aufzählung eine Limitierung des Erfindungsgegenstandes darstellt.

Als Wirtszellen kommen im Rahmen dieser Erfindung Prokaryonten in Frage, einschliesslich bakterieller Wirte, wie z.B. *A.tumefaciens, E.coli, S.typhimurium und Serratia marcescens*, sowie Cyanobakterien. Ferner können auch eukaryontische Wirte wie Hefen, mycelbildende Pilze und Pflanzenzellen im Rahmen dieser Erfindung verwendet werden.

Das Einspleissen der hybriden Genkonstruktion in einen geeigneten Klonierungsvektor erfolgt mit Hilfe von Standardmethoden wie sie z.B. bei Maniatis *et al* (1982) beschrieben sind.

Dabei wird in der Regel der Vektor und die einzuspleissende DNA Sequenz zunächst mit geeigneten Restriktions-Enzymen geschnitten. Geeignete Restriktionsenzyme sind z.B. solche, die Fragmente mit glatten Enden liefern, wie z.B. SmaI, HpaI und EcoRV, oder aber Enzyme, die kohäsive Enden bilden, wie z.B. EcoRI, SacI und BamHI.

Sowohl Fragmente mit glatten Enden wie auch solche mit kohäsiven Enden, die einander komplementär sind, können mit Hilfe geeigneter DNA-Ligasen wieder zu einem einheitlichen durchgehenden DNA-Molekül verknüpft werden.

EP 0 434 616 B1

Glatte Enden können auch durch Behandlung von DNA-Fragmenten, die überhängende kohäsive Enden aufweisen, mit dem Klenow-Fragment der *E.coli* DNA-Polymerase durch Auffüllen der Lücken mit den entsprechenden komplementären Nukleotiden hergestellt werden.

Anderseits lassen sich auch kohäsive Enden künstlich herstellen, z.B. durch Anfügen komplementärer homopolymerer Schwänze an die Enden einer gewünschten DNA-Sequenz sowie des geschnittenen Vektormoleküls unter Verwendung einer terminalen Desoxynukleotidyl-Transferase oder aber durch Anfügen synthetischer Oligonukleotid-Sequenzen (Linker), die eine Restriktionsschnittstelle tragen und anschliessendes Schneiden mit dem entsprechenden Enzym.

Der Klonierungsvektor und die mit diesem Vektor transformierte Wirtszelle werden in der Regel dazu verwendet, die Kopienzahl des Vektors zu erhöhen. Mit einer erhöhten Kopienzahl ist es möglich, den Vektor, der die hybride Genkonstruktion trägt, zu isolieren und z.B. für die Einschleusung der chimären Gensequenz in die pflanzliche Zelle vorzubereiten.

In einem weiteren Verfahrensschritt werden diese Plasmide dazu verwendet, die ein gewünschtes Genprodukt kodierenden Strukturgene oder aber nicht-kodierende DNA-Sequenzen mit regulatorischer Funktion, wie z.B. 'anti-sense' DNA, in die pflanzliche Zelle einzuschleusen und gegebenenfalls in das Pflanzengenom zu integrieren.

Ein weiterer Gegenstand der vorliegenden Erfindung betrifft daher die Herstellung von pflanzlichen Rezipienten-Zellen, welche besagtes Strukturgen oder sonstige wünschenswerte Gene bzw. Genfragmente oder sonstige nützliche DNA-Sequenzen in ihr Genom eingebaut enthalten.

Ebenfalls umfasst vom breiten Konzept dieser Erfindung sind somit transgene Pflanzen, insbesondere aber transgene fertile Pflanzen, die mit Hilfe des zuvor beschriebenen erfindungsgemässen Verfahrens transformiert worden sind sowie deren asexuelle und/oder sexuelle Nachkommenschaft, welche noch die durch die Transformation der Mutterpflanze bedingte(n) neue(n) und wünschenswerte(n) Eigenschaft(en) aufweisen.

Unter den Begriff der asexuellen und/oder sexuellen Nachkommenschaft transgener Pflanzen fallen definitionsgemäss im Rahmen dieser Erfindung somit auch alle Mutanten und Varianten die mit Hilfe bekannter Verfahren, wie z.B. durch Zellfusionen oder Mutantenselektion gewonnen werden können, und welche noch die charakteristischen Eigenschaften der transformierten Ausgangspflanze aufweisen, sowie sämtliche Kreuzungs- und Fusionsprodukte mit dem transformierten Pflanzenmaterial.

Ein weiterer Gegenstand dieser Erfindung betrifft das Vermehrungsgut transgener Pflanzen.

Unter Vermehrungsgut transgener Pflanzen soll im Rahmen dieser Erfindung jedes beliebige pflanzliche Material verstanden werden, das sich auf sexuellem oder asexuellem Wege bzw. *in−vivo* oder *in−vitro* vermehren lässt. Als besonders bevorzugt sind im Rahmen dieser Erfindung in erster Linie Protoplasten, Zellen, Kalli, Gewebe, Organe, Samen, Embryonen, Pollen, Eizellen, Zygoten anzusehen, sowie jedes beliebige andere Vermehrungsgut das von transgenen Pflanzen erhalten werden kann.

Pflanzenteile, wie z.B. Blüten, Stengel, Früchte, Blätter, Wurzeln, die von transgenen Pflanzen oder deren Nachkommen stammen, die zuvor mit Hilfe des erfindungsgemässen Verfahrens transformiert worden sind und die somit wenigstens zum Teil aus transgenen Zellen aufgebaut sind, sind ebenfalls Gegenstand dieser Erfindung.

Das erfindungsgemässe Verfahren eignet sich für die Transformation aller Pflanzen, insbesondere solchen der systematischen Gruppen *Angiospermae und Gymnospermae*.

Unter den *Gymnospermae* sind von besonderem Interesse die Pflanzen der Klasse *Coniferae*.

Unter den *Angiospermae* sind neben den Laubbäumen und Sträuchern von besonderem Interesse Pflanzen der Familien *Solanaceae, Cruciferae, Compositae, Liliaceae, Vitaceae, Chenopodiaceae, Rutaceae, Alliaceae, Amaryllidaceae, Asparagaceae, Orchidaceae, Palmae, Bromeliaceae, Rubiaceae, Theaceae, Musaceae, Malvaceae* oder *Gramineae* und der Ordnung *Leguminosae* und hier vor allem der Familie *Papilionaceae*. Bevorzugt sind Vertreter der Familien *Solanaceae, Cruciferae* und *Gramineae*.

Zu den Zielkulturen im Rahmen der vorliegenden Erfindung zählen beispielsweise auch jene, ausgewählt aus der Reihe: *Fragaria, Lotus, Medicago, Onobrychis, Trifolium, Trigonella, Vigna, Citrus, Linum, Geranium, Manihot, Daucus, Arabidopsis, Brassica, Raphanus, Sinapis, Atropa, Capsicum, Datura, Hyoscyamus, Lycopersion, Nicotiana, Solanum, Petunia, Digitalis, Majorana, Cichorium, Helianthus, Lactuca, Bromus, Gossypium, Asparagus, Antirrhinum, Hemerocallis, Nemesia, Pelargonium, Panicum, Pennisetum, Ranunculus, Senecio, Salpiglossis, Cucumis, Browallia, Glycine, Lolium, Zea, Triticum, Sorghum, Ipomoea, Passiflora, Cyclamen, Malus, Prunus, Rosa, Rubus, Populus, Santalum, Allium, Lilium, Narcissus, Ananas, Arachis, Phaseolus* und *Pisum*.

Besonders geeignet und damit bevorzugt für die Verwendung in dem erfindungsgemässen Verfahren sind embryonale und/oder meristematische Strukturen, die entweder isoliert oder im Verband der Gesamtpflanze vorliegen können. Da eine Regeneration ganzer Pflanzen ausgehend von embryonalen und/oder meristematischen Strukturen auch für eine Vielzahl monokotyler Pflanzen mittlerweile möglich ist, können im Rahmen der vorliegenden Erfindung auch die folgenden Pflanzen verwendet werden: *Lolium, Zea, Triticum, Sorghum, Saccharum, Bromus, Oryzae, Avena, Hordeum, Secale* und *Setaria*.

Die reifen Pflanzen, die aus transformierten Pflanzenzellen herangezogen wurden, werden zur Samenproduktion mit sich selbst gekreuzt. Einige der Samen enthalten die Gene, die für eine nützliche und wünschenswerte Eigenschaft kodieren in einem Verhältnis, das genau den etablierten Gesetzen der Vererbung gehorcht. Diese Samen können zur Produktion transgener Pflanzen verwendet werden.

Homozygote Linien können durch wiederholte Selbstbestäubung und Herstellung von Inzuchtlinien gewonnen werden. Diese Inzuchtlinien können dann wiederum zur Entwicklung von Hybriden verwendet werden. Bei diesem Verfahren wird eine Inzuchtlinie, die besagtes Fremdgen enthält mit einer anderen Inzuchtlinie zur Produktion gekreuzt.

Nach der allgemeinen Beschreibung der vorliegenden Erfindung soll nun zum besseren Verständnis auf spezifische Ausführungsbeispiele Bezug genommen werden, die zum Zwecke der Illustration in die Beschreibung mitaufgenommen werden, und die keinen limitierenden Charakter haben, es sei denn, es wird speziell darauf hingewiesen.

**Nichtlimitierende Ausführungsbeispiele**

**1. Herstellung der DNA/Goldpartikelsuspension**

**1.1 Plasmid**

Im Rahmen der nachfolgenden Transformationsexperimente wird das Plasmid pBI221 verwendet, welches ein $\beta$-Glucuronidase-Enzym exprimiert. Dieses Plasmid ist bei CLONTECH, Palo Acto CA, USA sowie bei der GENOFIT in Genf erhältlich. Detaillierte Angaben zu dessen Konstruktion und Zusammensetzung sind in einer Publikation von Jefferson et al (1986) enthalten.

Für die nachfolgend im Detail beschriebenen Transformationsexperimente wird das Plasmid pBI221 in einer 'super-coiled' Konfiguration verwendet und zwar in einer Konzentration von ca. 1.25 $\mu$g DNA/$\mu$l $H_2O$. Die DNA-Lösung wird bei -20° C gelagert und erst kurz vor Gebrauch aufgetaut.

**1.2 Herstellung der Goldpartikel**

Zu 10 ml einer wässrigen Lösung von 1% Tetrachlorgoldsäure [$H(AuCl_2) \cdot 3H_2O$; Merck No. 1582] werden in einem spitz auslaufenden 15 ml Plastik-Zentrifugenröhrchen 200 $\mu$l eines photographischen Entwicklers [z.B. 'Rondial', unverdünnt (AGFA)] rasch (< 10 s) zupipettiert. Der gesamte Ansatz wird anschliessend einmal kurz geschüttelt. Nach einer Inkubationszeit von 30 s bei Raumtemperatur wird die Reduktionsreaktion durch Zugabe von 2 ml photographischem Fixierer [z.B. 'Ilfospeed', käufliche Stammlö-sung 1+4 mit $H_2O$ dest. verdünnt; Ilford Foto AG] gestoppt.

Die Suspension wird anschliessend 5 Minuten bei 2.2 k x g zentrifugiert [Ausschwingrotor, z.B. Christ Labofuge, HERAEUS], der Überstand verworfen und der verbleibende Rückstand in 1.5 ml $H_2O$ resuspen-diert und in ein Eppendorfgefäss überführt. Mit einer zweiten Zentrifugation (3 Minuten bei 10 k x g, Eppendorizentrifuge, Festwinkelrotor) wird das restliche Fixiersalz ausgewaschen. Der Rückstand wird in 1.1 ml $H_2O$ resuspendiert und anschliessend in zwei Aliquots bei 1 bar und 120° C autoklaviert.

Die autoklavierte Suspension enthät 0.5 (±0.1) x $10^9$ Gold- Partikel/ml. Der mittlere Partikeldurchmesser beträgt 1.6 $\mu$m ± 0.01 $\mu$m (Standardfehler des Mittelwertes) mit einem maximalen Partikeldurchmesser von 2.5 $\mu$m (ein Partikel aus ca. 50). Die autoklavierte Suspension wird im Kühlschrank aufbewahrt und erst kurz vor Gebrauch resuspendiert.

**1.3 DNA/Goldpartikel-Gemisch**

Unmittelbar vor Gebrauch werden die folgenden Komponenten zusammenpipettiert:

3 $\mu$l pBI221 'super-coiled' DNA
6 $\mu$l Goldsuspension
1 $\mu$l 100 mM Tris-HCl, pH 7.0
1 $\mu$l 1 mM Na-EDTA

Als Kontrolle dient eine Lösung gleicher Zusammensetzung, wobei jedoch anstelle der pBI221 Plasmid DNA Wasser verwendet wird. In einem der durchgeführten Experimente wird die Goldsuspension mit 1+9 mit $H_2O$ verdünnt.

14

## 2. Vorbereitung des zu transformierenden Pflanzenmaterials

### 2.1 Maisembryonen

Die Embryonen werden kurz vor der Reife aus noch unreifen Maiskörnern freipräpariert und auf einem Medium nach Murashige und Skoog (1962) [vergl. Tabelle 1] in kleinen Petrischalen abgelegt.

### 2.2 Montage der Maisembryonen

2% Agarose mit 10 mM $CaCl_2$ wird auf sterile Thermanoxplättchen [22 x 60 mm, Miles Laboratories, Naperville, USA] so ausgegossen, dass eine gleichmässige, ca. 1 mm starke Agaroseschicht entsteht Die Embryonen werden anschliessend mit Hilfe eines Tropfens (< 10 $\mu$l) 4% Alginat [z.B. Filter-sterilisiertes Natrium-Alginat, Fluka No. 71238] auf der Agarose montiert, wobei darauf zu achten ist, dass das Scutellum nach oben gerichtet ist. Das Alginat wird vor dem Beschiessen ca. 2-3 Stunden in einer feuchten Kammer gehärtet.

Die Montage der Embryonen kann auf verschiedene Art und Weise durchgeführt werden. Bevorzugt ist ein Verfahren, bei dem die Embryonen mit Hilfe einer Drahtschlinge aus ihrer Nährlösung entnommen und, nach Beseitigung des vorhandenen Flüssigkeitsüberschusses mit Hilfe eines Filterpapiers, unter dem Stereomikroskop auf die Agaroseunterlage aufgeklebt werden. Der Alginatüberschuss wird mit einem sterilen Filterpapier abgesaugt. Anschliessend lässt man das Alginat 2-3 Stunden in einer feuchten Kammer aushärten. Auf eine Agaroseunterlage können auf diese Weise bis zu 40 Embryonen und mehr nebeneinander montiert werden.

Unmittelbar vor dem Beschiessen wird die Agaroseunterlage mit mit einem Sklapell zerschnitten, so dass jeder Embryo mitsamt der Agaroseunterlage einzeln auf einen sterilen Objekttäger aufgesetzt werden kann. Dieser wird dann an die Probenkammer anstelle der Vorrichtung 23 der zuvor im Detail beschriebenen Apparatur eingebracht. Durch das Anlegen eines Wasserstrahlpumpenvakuums wird der Objektträger mit dem aufgesetzten Embryo an der Apparatur angesaugt, wobei er aber verschiebbar bleibt.

## 3. Schiessvorgang

### 3.1 Zielen

Ein fest markiertes Fadenkreuz markiert die Verlängerung der Schussachse. Durch Peilen vom Fadenkreuz auf die Mündung und verschieben des Objektträgers wird der Embryo in die Schussachse gebracht.

### 3.2 Laden der Kanüle

Zum Laden der zuvor im Detail beschriebenen Kanüle wird diese vorübergehend aus der Druckkammer 1 entfernt Das Paraffin [MERCK No. 7174], das als Verdrängungsflüssigkeit im Dosieraggregat 45-49 fungiert, wird vorgetrieben bis der Meniskus an der Kanülenöffnung erscheint. Ein ca. 1 $\mu$l umfassender Tropfen der fertigen Suspension wird mit der Pipette aufgesetzt und mit Hilfe des Dosieraggregats 45-49 in die Kanüle hereingezogen. Die Kanüle fasst 2 $\mu$l bis 3 $\mu$l Suspension. Wenn der letzte der auf die zuvor beschriebene Weise aufgesetzten Tropfen ganz in der Kanüle verschwunden ist, wird der Suspensionsmeniskus soweit vorgeschoben, bis er mit dem Kanülenende bündig ist und die Kanüle wieder in die Druckkammer 1 eingeschraubt.

### 3.3 Einschiessen der DNA-/Goldpartikel-haltigen Suspension in die Maisembryonen

Für das Einschiessen der DNA- und Goldpartikel-haltigen Suspension in die Maisembryonen werden die folgenden Paramter an der erfindungsgemässen Vorrichtung eingestellt:
- Evakuieren der Probenkammer 2 mittels einer Wasserstrahlpumpe (ca. 500 mbar).
- $CO_2$-Druck beim Schuss: 57 bar bei 20° C.
- Dauer des einzelnen Schusses: 2 ms
- Arbeitsabstand (Mündung 42a der Kanüle 42-Probe): 10 mm
- Kapillare (17): Glaskapillare mit einer Länge von 10 mm und einem Durchmesser von 300 $\mu$m.
- Winkel $\alpha$ zwischen Kanüle und der Vertikalen: 90°
- Anzahl Schüsse pro Embryo: 1 oder 5 (gemäss Tabelle 2)

- Volumen Ladung/Embryo: 0.2 $\mu$l
- Modus:
    a) 1 Schuss:     0,2 $\mu$l Suspension (= Ladung) → 1 Schuss
    b) 5 Schüsse:     0,1 $\mu$l Suspension (= Ladung) → 2 Schüsse
                         0,1 $\mu$l Suspension (= Ladung) → 3 Schüsse

## 4. Kultivierung der transformierten Embryonen

Nach dem Beschuss mit der DNA-/Partikel-haltigen Suspension wird der Embryo aus dem Alginat herausgelöst und in einem Medium nach Murashige und Skoog (vergl. Tabelle 1) kultiviert. Dabei werden je 5 bis 7 Embryonen auf 0.5 ml Medium in Vertiefungen von Gewebekulturplatten (mit Deckel) [Costar-Platten mit 24 Vertiefungen (Durchmesser: 16 mm); COSTAR, Cambridge, USA] gegeben. Es werden vorteilhafterweise nur die inneren Vertiefungen mit Embryonen belegt, während die äusseren mit je 1 ml $H_2O$ gefüllt werden, sodass das gesamte System als feuchte Kammer dient. Die Embryonen werden für einen Zeitraum von 48 Stunden bei Zimmertemperatur und Tageslicht [unterstütz durch Kunstlicht: OSRAM L 36 W/77 Fluora in einem Abstand von 15 cm] kultiviert.

## 5. GUS ($\beta$-Glucuronidas)- Nachweis

Der Nachweis einer vorübergehenden Expression ('transient expression') des $\beta$-Gucuronidase-Gens erfolgt mit Hilfe einer Modifikation des bei Mendel *et al* (1989) beschriebenen Verfahrens.

Reaktions-Lösungen:

Lösung (a):

| Einzelkomponente | Stammlösung | Volumen | Endkonzentration |
|---|---|---|---|
| K/Na Phosphatpuffer, (pH 7.0) | 0.5 M | 1000 $\mu$l | 0.1 M |
| Na-EDTA | 100 mM | 500 $\mu$l | 10 mM |
| Triton-X | 1.0 % | 500 $\mu$l | 0.1 % |
| Kaliumferricyanid II | 50 mM | 500 $\mu$l | 5 mM |
| Kaliumferricyanid III | 50 mM | 500 $\mu$l | 5 mM |
| $H_2O$ | | 2000 $\mu$l | |

Lösung (b):

Substratlösung bestehend aus: 5mg 5-Brom-4-chlor-indol-3-yl-$\beta$-D-glucuronsäure [Clontech Laboratories, Palo Alto, USA] gelöst in 50 $\mu$l Dimethylformamid. Diese Substratlösung wird stets frisch angesetzt.

Lösung (c):

Die Lösungen (a) und (b) werden miteinander vermischt und sofort für den GUS-Nachweis verwendet. Dies führt in Abweichung von Mendel *et al* (1989) zu einer Substratkonzentration von 0.1%.

Für den GUS-Nachweis wird zunächst die die Embryonen umgebende Nährlösung mit Hilfe einer Pipette aus den Vertiefungen der Gewebekulturplatten entfernt und durch jeweils 0.5 ml Lösung (c) ersetzt (ausreichend für jeweils 5 bis 7 Embryonen). Die geschlossene Kulturplatte wird dann 24 Stunden bei 37°C im Brutschrank inkubiert.

Nach 24 Stunden sind blaue Flecken auf den Embryonen zu erkennen, die das Vorliegen einer GUS-Aktivität in den embryonalen Zellen anzeigen. Die Flecken werden unter dem Stereomikroskop ausgezählt. Die Tabelle 2 gibt die Ergebnisse eines repräsentativen Versuches wieder, wobei bei positiven Embryonen bis zu 14 Flecken gezählt werden konnten. Die Kontrollen waren dagegen ausnahmslos negativ.

## 6. Transformation von Bakterienzellen

### 6.1. Transformation von *E. coli* Zellen

Escherichia coli Zellen des Stammes JM101 [Yanisch-Perron *et al* (1985)] werden in einem der üblicherweise verwendeten Medien, z.B. einem LM-Medium [Miller JH (1977)], auf Nitrozellulosefiltern kultiviert. Die Filter werden in Quadrate von ca. 0.5 cm zerschnitten und ein Agarosestückchen [2%] gleicher Grösse aufgelegt.

Dies wird anschliessend in die Probenkammer eingebracht, wie in Beispiel 2.2. im Detail beschrieben. Für das Einschiessen der pUC18 [Plasmid-DNA- und Goldpartikel-haltigen Suspension in die Bakterienzellen werden die folgenden Paramter, die während des gesamten Experiments unverändert bleiben, an der erfindungsgemässen Vorrichtung eingestellt:

- Evakuieren der Probenkammer 2 mittels einer Wasserstrahlpumpe (ca. 900 mbar).
- $CO_2$-Druck beim Schuss: 55 bar bei 20° C.
- Dauer des einzelnen Schusses: 2 ms
- Arbeitsabstand (Mündung 42a der Kanüle 42-Probe): siehe Tabelle 1
- Kapillare (17): Glaskapillare mit einer Länge von 10 mm und einem Durchmesser von 200 $\mu$m.
- Winkel $\alpha$ zwischen Kanüle und der Vertikalen: 45°
- Modus: Die Schüsse werden an drei verschiedene Stellen des Filterquadrates plaziert, wobei pro Stelle jeweils drei Schüsse [20 nl DNA/Partikel Suspension pro Schuss] abgegeben werden.

Die DNA-Konzentration sowie die Konzentration der Partikel in der Suspension, desweiteren die Grösse der Partikel und der Arbeitsabstand werden gemäss den Angaben in Tabelle 1 im Verlaufe des Experiments variiert.

Nach Beendigung des Schiessvorganges werden die Proben 1 Stunde in einem Flüssigmedium [LM-Medium] inkubiert um die Bakterien von den Filtern zu lösen. Danach wird die bakterielle Suspension auf einem festen LM-Selektionsmedium ausplattiert, das mit 100 $\mu$g/ml Ampicillin und 1.5% Agar angereichert ist. Nach einer 24 stündigen Inkubation auf besagtem Medium werden die Kolonien gezählt [siehe Tabelle 1]. Eine Auswahl positiver Kolonien wird auf seinen Plasmidgehalt hin untersucht.

Plasmid-DNA wird von den Bakterien isoliert und auf einem Gel aufgetrennt. Die Grösse der DNA wird bestimmt anhand eines Molekulargewichts-Markers [BRL; Bethseda Research Laboratories Life Technolgies; # 5615 SA/SB] sowie eines Standard-Plasmids [pUC18 geschnitten mit BamHI; 2.7 Kbp]. Alle getesteten positiven Kolonien erhielten das korrekte pUC18 plasmid.

Die Kontrollen werden nur mit den Partikeln, aber ohne DNA beschossen oder sie werden ohne vorheriges Beschiessen in einem Überschuss von DNA inkubiert. Sämtliche Kontrollen waren negativ.

Da der Durchmesser der Bakterien kleiner ist als die Partikelgrösse, ist es unwahrscheinlich, dass eine Zelle, in die ein solcher Partikel eindringt, überlebensfähig ist. Man kann daher davon ausgehen, dass im Falle eines transgenen Ereignisse der Partikel die Zellwand sowie die vorhandenen Membranen nur soweit verletzt hat, dass es den DNA Molekülen möglich ist durch kleine, reversible Öffnungen in die Zelle zu gelangen, wahrend der Goldpartikel selbst ausserhalb der Zelle verbleibt.

Bei den hier beschriebenen Verfahren zur Kultivierung von *E. coli* sowie zur Isolierung und Charakterisierung von *E. coli* Plasmiden handelt es sich um Standardverfahren, die dem Fachmann auf diesem Gebiet bestens bekannt sind und die z.B. bei Maniatis *et al* (1982) im Detail beschrieben sind.

## 6. Transformation von Tabakzellen aus Mikrokalli mit dem Neomycinphosphotransferase-Gen

Tabak-Kallus wird in Analogie zu den in den Beispielen 1 bis 5 beschriebenen Verfahren mit dem Neomycinphosphotransferase-Gen transformiert.

Die stabile Integration des Gens ins pflanzliche Genom konnte anhand von Southern Blot Daten verifiziert werden.

## 7. Tabellen

Tabelle 1

| Hormon-freies Medium nach Murashige und Skoog (1962) [MSO-2 Medium; pH 5.8] | |
|---|---|
| Einzelkomponente | Konzentration [mgll] |
| $CaCl_2$ x $2H_2O$ | 440 |
| $NH_4NO_3$ | 1650 |
| $KNO_3$ | 1900 |
| KI | 0.83 |
| $CoCl_2$ x $6H_2O$ | 0.025 |
| $KH_2PO_4$ | 170 |
| $H_3BO_3$ | 6.2 |
| $Na_2MoO_4$ x $2H_2O$ | 0.25 |
| $MgSO_4$ x $7H_2O$ | 370 |
| $MnSO_4$ x $4H_2O$ | 22.3 |
| $CuSO_4$ x $5H_2O$ | 0.025 |
| $ZnSO_4$ x $7H_2O$ | 8.6 |
| $FeSO_4$ x $7H_2O$ | 27.85 |
| $Na_2$ EDTA | 37.25 |
| Glycin | 2.0 |
| Inosit | 100 |
| Nikotinsäure | 0.5 |
| Pyridoxin HCl | 0.5 |
| Thiamin HCl | 0.1 |
| Saccharose | 20.000 |

Tabelle 2: Ergebnisse der in den Beispielen 1 bis 5 beschriebenen Transformations-experimente

| | | Anzahl der behandelten Embryonen | Anzahl der Transformanten | Transformations-frequenz [ % ] |
|---|---|---|---|---|
| pBI221 | 1) | 15 | 1 | 6 |
| | 2) | 18 | 9 | 50 |
| | 3) | 23 | 5 | 22 |
| Kontrolle | 1) | 8 | 0 | 0 |
| | 2) | 11 | 0 | 0 |
| | 3) | 20 | 0 | 0 |

In Tabelle 2 sind drei unabhängig voneinander durchgeführte Experimente wiedergegeben:

1) In Experiment 1 beträgt die Partikelkonzentration in der Suspension ca. $0.3 \times 10^6$ Part/$\mu$l In diesem Fall wurde nur 1 mal geschossen mit einem Suspensionsvolumen von 0.2 $\mu$l

2) In Experiment 2 beträgt die Partikelkonzentration in der Suspension ca. $0.3 \times 10^6$ Part/$\mu$l Ein Suspensionsvolumen von $2 \times 0.1$ $\mu$l wird auf 5 Schüsse verteilt.

3) In Experiment 3 beträgt die Partikelkonzentration in der Suspension ca. $0.3 \times 10^5$ Part/$\mu$l Ein Suspensionsvolumen von $2 \times 0.1$ $\mu$l wird auf 5 Schüsse verteilt.

Tabelle 3: Resultate der in Beispiel 6 beschriebenen Transformationsexperimente

**Tabelle 3**

| Parameter | | | | | | | Ergebnisse | | |
|---|---|---|---|---|---|---|---|---|---|
| # | a | b | c | d | e | f | g | h | i |
| 13/1 | 7 | 63 | 0.5 | 1.0 | 1x | 10 | 0 | 0 | 0 |
| 13/2 | 9 | 72 | 1.5 | 1.5 | 10x | 7 | 6 | 50 | 2 |
| 13/3 | 18 | 162 | 5.0 | 1.5 | 10x | 3 | 42 | 83 | 11 |

Legende:

\#: Zahl der Experimente

a: Zahl der Filterquadrate (= Proben, 9 Schüsse je Probe)

b: Gesamtzahl der pro Experiment abgegebenen Schüsse

c: Konzentration des in der DNA/Partikel-Suspension vorliegenden Plasmids in $\mu$g/$\mu$l.

d: Partikeldurchmesser in $\mu$m

e: Partikelkonzentration, angegeben als arbiträre Grau-Stufen

f: Arbeitsabstand in mm. Dabei handelt es sich um die Distanz zwischen der Spitze der Glaskapillare und der Probenoberfläche.

g: Zahl positiver Kolonien

h: Anzahl derjenigen Proben in %, die positive Kolonien enthalten.

i: Maximale Zahl positiver Kolonien pro Probe

Literaturverzeichnis

1. Birk et al, Biochim. Biophys. Acta, 67: 326-238, 1963
2. Cashmore A, "Genetic Engineering of Plants, an Agricultural Perspective", Plenum, New York, 1983, Seite 29-38
3. Chilton M-D, Scientific American, 248: 50-59, 1983
4. Christon et al, Plant Physiol., 87: 671-674, 1988
5. De La Pena et al, Nature, 325: 274-276, 1987
6. Frank G et al, Cell, 21: 285-294, 1980
7. Gardner RC et al, Nucl. Acids Res., 9: 2871-2888, 1981
8. Grimsley NH et al, Nature, 325: 177-179, 1987
9. Hernalsteens JP et al, EMBO J., 3: 3039-3041, 1984
10. Hilder VA et al, Nature, 330: 160-163, 1987
11. Hohn T et al, "Molecular Biology of Plant Tumors", Academic Press, Seite 549-560, 1982

12. Hooykaas-Van-Slogteren et al, Nature, 311: 763-764, 1984

13. Howard et al, Planta, 170: 535, 1987

14. Jefferson et al, Proc. Natl. Acad. Sci., USA, 83: 8447-8451, 1986

15. Klein et al, Proc. Natl. Acad. Sci., USA, 85: 4305-4309, 1988

16. Maniatis et al, Molecular Cloning, Cold Spring Harbor Laboratories, 1982

17. Mendel et al, Theor. Appl. Genet., 78: 31-34, 1989

18. Miller JH, Experiments in Molecular Genetics, 1972, Cold Spring Harbor Laboratory, New York, 3rd ed., 1977, p. 433

19. Morelli et al, Nature, 315: 200, 1985

20. Morikawa H und Yamada Y, Plant Cell Physiol., 26: 229-236, 1985

21. Murashige und Skoog, Physiol. Plant., 15: 473, 1962

22. Nomura K und Komamine A, Plant Sci., 44: 53-58, 1986

23. Paszkowski J et al, EMBO J, 3: 2717-2722, 1984

24. Potrykus I et al, "Direct Gene Transfer to Protoplasts: An Efficient and Generally Applicable Method for Stable Alterations of Plant Genoms" in: Freeling M (ed.), Plant Genetics, A.R. Liss Inc., New York, pp. 181-199, 1986

25. Spena et al, EMBO J, 4: 2736, 1985

26. Steinbiss HH und Stable P, Protoplasma, 116: 223-227, 1983

27. Vieira and Messing, Gene 19: 259-268, 1982

28. Yanisch-Perton et al, Gene 33: 103, 1985

29. EP-A 0 270 356

**Patentansprüche**

1. Verfahren zur genetischen Transformation von Zellen, insbesondere Pflanzenzellen durch Beschuss der Zellen mit DNA mit sich führenden Mikroprojektilen, insbesondere Gold- oder Wolframpartikeln in Mikrometergrösse mit einem Impuls so, dass die Mikroprojektile in die zu transformierenden Zellen eindringen oder diese zumindest soweit verletzten, dass die DNA in die Zelle vordringen kann, wobei die Mikroprojektile in einer Lösung der DNA suspendiert werden und ein definiertes Volumen dieser Suspension bereitgestellt wird, dadurch gekennzeichnet, dass das definierte Volumen der Suspension innerhalb einer Druckkammer (1) bereitgestellt wird und dass die Mikroprojektile innerhalb der Druckkammer durch einen Druckstoss aus dem bereitgestellten definierten Volumen dieser Suspension heraus zusammen mit der Lösung fein zerstäubt und aus der Druckkammer heraus in Richtung auf die zu transformierenden Zellen beschleunigt werden.

2. Verfahren nach Anspruch 1, dadurch gekennzeichnet, dass der bei der Zerstäubung entstehende, die Mikroprojektile und an diesen anhaftende DNA enthaltende Nebel durch eine auf die zu transformierenden Zellen ausgerichtete Restriktion (17) in Form einer Blende oder einer Kapillare gepresst und dadurch auf die Zellen hin beschleunigt und fokussiert wird.

3. Verfahren nach Anspruch 1 oder 2, dadurch gekennzeichnet, dass die zu transformierenden Zellen zumindest während ihres Beschusses mit den Mikroprojektilen in einem zumindest teilweise evakuierten Raum (2) angeordnet werden.

4. Verfahren nach einem der Ansprüche 1-3, dadurch gekennzeichnet, dass jeweils ein Tropfen (T) DNA-Lösung mit darin suspendierten Mikroprojektilen bereitgestellt und dieser Tropfen dann durch den Druckstoss zerstäubt wird.

5. Verfahren nach Anspruch 4, dadurch gekennzeichnet, dass der Tropfen (T) in einer geraden Linie (A) mit der Restriktion (17) und den Zellen angeordnet wird und dass die Hauptausbreitungsrichtung des Druckstosses kollinear mit dieser Linie verlaufend gewählt wird.

6. Verfahren nach einem der Ansprüche 1-5, dadurch gekennzeichnet, dass als Mikroprojektile Partikeln, insbesondere Goldpartikeln im wesentlichen einheitlicher Grösse im Bereich von 20 nm bis 5 $\mu$m, vorzugsweise 1,2-1,5 $\mu$m Durchmesser verwendet werden.

7. Verfahren nach Anspruch 4, dadurch gekennzeichnet, dass das Volumen eines Tropfens (T) im Bereich von 10-500 nl, vorzugsweise etwa 50-150 nl gewählt wird.

8. Verfahren nach den Ansprüchen 2 und 4, dadurch gekennzeichnet, dass der Abstand zwischen dem Tropfen (T) und der Restriktion (17) vorzugsweise im Bereich von etwa 5-20 mm so gross gewählt wird, dass kein geschlossener Flüssigkeitfaden in die Restriktion eintreten kann.

9. Verfahren nach Anspruch 4, dadurch gekennzeichnet, dass der Tropfen (T) am Austrittsende (42a) einer zur Vertikalen (V) geneigten Kanüle (42) erzeugt wird, wobei der Winkel (α) der Kanüle zur Vertikalen (V) in Abhängigkeit von der Grösse der Mikroprojektile und der Viskosität der die suspendierten Mikroprojektile enthaltenden DNA-Lösung so gewählt wird, dass die Konzentration der suspendierten Mikroprojektile in aufeinanderfolgenden Tropfen im wesentlichen konstant bleibt.

10. Verfahren nach Anspruch 2, dadurch gekennzeichnet, dass die Restriktion (17) einen freien Durchflussdurchmesser im Bereich von 20-300 $\mu$m aufweist.

11. Verfahren nach Anspruch 2, dadurch gekennzeichnet, dass als Restriktion eine Kapillare (17) mit einem Innendurchmesser von 10 $\mu$m bis 500 $\mu$m und mit einer Länge von 1-20 mm verwendet wird.

12. Verfahren nach einem der Ansprüche 1-11, dadurch gekennzeichnet, dass der Druckstoss im Bereich von 2-10000 bar, vorzugsweise im Bereich von einigen zehn bar bis einigen hundert bar gewählt wird.

13. Verfahren nach einem der Ansprüche 1-12, dadurch gekennzeichnet, dass bei einer DNA-Konzentration im Bereich von einigen Zehntel $\mu$g pro $\mu$l gearbeitet wird.

14. Vorrichtung zum Einbringen von DNA mit sich führenden Partikeln in insbesondere pflanzliche Zellen, insbesondere zur genetischen Transformation dieser Zellen, mit einem Träger (25) für die Zellen, mit Mitteln (4) zum Bereitstellen eines definierten Volumens einer Partikeln und DNA enthaltenden Suspension und mit Mitteln (3) zur Beschleunigung der Partikeln zum Zellenträger hin, dadurch gekennzeichnet, dass eine Druckkammer (1) vorgesehen ist, dass die Dosiermittel (4) das definierte Volumen (T) innerhalb der Druckkammer (1) bereitstellen und dass die Mittel (3) zur Beschleunigung der Partikeln dazu ausgebildet sind, dieses Volumen (T) innerhalb der Druckkammer (1) in einen feinen Nebel zu zerstäuben und diesen Nebel aus der Druckkammer (1) heraus zum Zellenträger (25) hin zu beschleunigen.

15. Vorrichtung nach Anspruch 14, dadurch gekennzeichnet, dass die Druckkammer (1) im wesentlichen langgestreckt ist, dass an einem Ende der Druckkammer eine Einrichtung (31) zur Erzeugung eines Druckstosses angeordnet ist und das andere Ende in eine Restriktion (17) ausmündet, dass der Zellenträger (25) in der Verlängerung der Druckkammer (1) mit der Restriktion (17) fluchtend im Abstand (b) zu dieser angeordnet ist, und dass die Dosiermittel (4) eine soweit in die Druckkammer (1) hineinragende Kanüle (42) aufweisen, dass die Mündung (42a) der Kanüle, die Restriktion (17) und der Zellenträger (25) im wesentlichen auf einer geraden, zur Längsachse (A) der Druckkammer (1) parallelen oder mit dieser zusammenfallenden Linie liegen.

16. Vorrichtung nach Anspruch 15, dadurch gekennzeichnet, dass die Dosiermittel (4) eine die Kanüle (42) speisende einstellbare Dosiereinrichtung (44-49) umfassen, welche dazu befähigt ist, an der Mündung (42a) der Kanüle (42) gleichbleibende Suspensions-Tropfen (T) definierten einstellbaren Volumens entstehen zu lassen.

17. Vorrichtung nach Anspruch 15 oder 16, dadurch gekennzeichnet, dass der Winkel (α), unter dem die Kanüle (42) in die Druckkammer (1) ausmündet, verstellbar ist.

18. Vorrichtung nach einem der Ansprüche 15-17, dadurch gekennzeichnet, dass die Druckkammer (1) am restriktionsseitigen Ende sich gegen die Restriktion (17) hin verjüngend ausgebildet ist.

19. Vorrichtung nach Anspruch 17, dadurch gekennzeichnet, dass die Druckkammer (1) um ihre Längsachse (A) wenigstens begrenzt verdrehbar angeordnet ist.

20. Vorrichtung nach einem der Ansprüche 15-19, dadurch gekennzeichnet, dass sie eine mit einem Anschluss (28) für eine Saugquelle (5) versehene, wenigstens teilweise evakuierbare Probenkammer (2) aufweist, in welche die Restriktion (17) mündet und in welcher der Zellenträger (25) angeordnet ist.

21. Vorrichtung nach Anspruch 20, dadurch gekennzeichnet, dass der Zellenträger (25) innerhalb der Probenkammer (2) wenigstens quer zur Längsrichtung (A) der Druckkammer (1) verstellbar angeordnet ist.

22. Vorrichtung nach Anspruch 21, dadurch gekennzeichnet, dass die Probenkammer (2) einen abnehmbaren Teil (23) aufweist, und dass der Zellenträger (25) zusammen mit Justiermitteln (27) zu seiner örtlichen Verstellung in diesem abnehmbaren Teil (23) der Probenkammer (2) angeordnet ist.

23. Vorrichtung nach einem der Ansprüche 15-22, dadurch gekennzeichnet, dass in der Kanüle (42) oder in einer Zuleitung zu dieser ein Rückschlagventil (43) vorgesehen ist.

24. Vorrichtung nach einem der Ansprüche 15-23, dadurch gekennzeichnet, dass die Einrichtung zur Erzeugung eines Druckstosses ein druckluft- oder druckgasbetriebenes pistolenartiges Gerät (31) ist.

25. Vorrichtung nach einem der Ansprüche 15-24, dadurch gekennzeichnet, dass die Restriktion (17) eine Blende oder eine Kapillare ist.

26. Vorrichtung nach Anspruch 25, dadurch gekennzeichnet, dass der freie Durchmesser der Blende etwa 20-300 $\mu$m beträgt.

27. Vorrichtung nach Anspruch 25, dadurch gekennzeichnet, dass der Innendurchmesser der Kapillare (17) etwa 10-500 $\mu$m und die Länge der Kapillare (17) etwa 1-20 mm beträgt.

28. Vorrichtung nach einem der Ansprüche 15-27, dadurch gekennzeichnet, dass der Abstand der Mündung (42a) der Kanüle (42) von der Restriktion (17) etwa 5-20 mm beträgt.

29. Verfahren gemäss Anspruch 1, dadurch gekennzeichnet, dass besagte Zellen als Bestandteil einer höher organisierten pflanzlichen Einheit vorliegen.

30. Verfahren gemäss Anspruch 29, dadurch gekennzeichnet, dass es sich um einen wenig-zelligen Gewebeverband handelt.

31. Verfahren gemäss Anspruch 30, dadurch gekennzeichnet, dass es sich um einen meristematischen Gewebeverband handelt.

32. Verfahren gemäss einem der Ansprüche 1-13, dadurch gekennzeichnet, dass es sich bei den zu transformierenden Zellen um pflanzliche Pro-Embryonen oder pflanzliche Embryonen oder embryogene pflanzliche Zellkulturen handelt.

33. Verfahren zur Herstellung einer transgenen Pflanze, dadurch gekennzeichnet, dass man eine gemäss dem Verfahren nach einem der Ansprüche 1-13 oder 29-32 hergestellte Pflanzenzelle zu einer ganzen transgenen Pflanze regeneriert.

34. Verfahren gemäss einem der Ansprüche 1 bis 13, dadurch gekennzeichnet, dass es sich bei besagter Zelle um eine prokaryontische Zelle handelt.

35. Verfahren gemäss einem der Ansprüche 1 bis 13, dadurch gekennzeichnet, dass es sich bei besagter Zelle um eine Bakterienzelle handelt.

## Claims

1. A process for the genetic transformation of cells, in particular plant cells, by bombarding the cells with DNA-carrying microprojectiles, especially gold or tungsten particles of micrometre size, with an impulse such that the microprojectiles penetrate or at least wound the cells to be transformed in a way sufficient to enable the DNA to enter the cell, the microprojectiles being suspended in a solution of the DNA and a defined volume of that suspension being prepared, wherein the defined volume of the suspension is prepared inside a pressure chamber (1) and, inside the pressure chamber, the microprojectiles are finely atomised together with the solution by a pulse of pressure out of the prepared defined volume of the suspension and are accelerated out of the pressure chamber in the direction of the cells to be transformed.

2. A process according to claim 1, wherein the mist generated by the atomisation and containing the microprojectiles and DNA adhering thereto is compressed by a constriction (17), in the form of a diaphragm or capillary, aligned in the direction of the cells to be transformed and is thus accelerated towards and focused on the cells.

3. A process according to either claim 1 or claim 2, wherein the cells to be transformed are located in an at least partially evacuated chamber (2) at least during their bombardment with the microprojectiles.

4. A process according to any one of claims 1 to 3, wherein one drop (T) of the DNA solution with the microprojectiles suspended therein is prepared and that drop is then atomised by the pulse of pressure.

5. A process according to claim 4, wherein the drop (T) is arranged in a straight line (A) with the constriction (17) and the cells and the principal direction of propagation of the pulse of pressure is selected to be collinear with that line.

6. A process according to any one of claims 1 to 5, wherein particles, especially gold particles of an essentially uniform size in a diameter range of 20 nm to 5 $\mu$m, preferably 1.2 to 1.5 $\mu$m, are used as the microprojectiles.

7. A process according to claim 4, wherein the volume of a drop (T) is in the range of 10 to 500 nl, preferably about 50 to 150 nl.

8. A process according to claim 2 and claim 4, wherein the distance between the drop (T) and the constriction (17) is chosen, preferably within the range of about 5 - 20 mm, to be such that no unbroken liquid filament may enter the constriction.

9. A process according to claim 4, wherein the drop (T) is produced at the outlet end (42a) of a cannula (42) inclined relative to the vertical (V), the angle ($\alpha$) of the cannula to the vertical (V) being chosen as a function of the microprojectile size and the viscosity of the DNA solution containing the suspended microprojectiles to be such that the concentration of the suspended microprojectiles remains essentially constant in successive drops.

10. A process according to claim 2, wherein the constriction (17) has a free flow passage diameter in the range of 20 - 300 $\mu$m.

11. A process according to claim 2, wherein as the constriction a capillary (17) with an internal diameter of 10 $\mu$m to 500 $\mu$m and a length of 1 - 20 mm is used.

12. A process according to any one of claims 1 to 11, wherein the pulse of pressure chosen is in the range of 2 to 10 000 bar, preferably in the range of several tens of bars to several hundreds of bars.

13. A process according to any one of claims 1 to 12, wherein a DNA concentration in the range of several tenths $\mu$g per $\mu$l is used.

14. An apparatus for the introduction of DNA-carrying particles into cells, in particular plant cells, especially for the genetic transformation of those cells, comprising a holder (25) for the cells, means (4) for preparing a defined volume of a suspension containing particles and DNA and means (3) for accelerating the particles towards the cell holder, wherein a pressure chamber (1) is provided, the metering means (4) prepares the defined volume (T) inside the pressure chamber (1) and the means (3) for accelerating the particles is designed to atomise that volume (T) inside the pressure chamber (1) into a fine mist and to accelerate that mist out of the pressure chamber (1) and towards the cell holder (25).

15. An apparatus according to claim 14, wherein the pressure chamber (1) is essentially elongate, a device (31) for generating a pulse of pressure is arranged at one end of the pressure chamber and the other end opens into a constriction (17), the cell holder (25) is located in an extension of the pressure chamber (1) aligned with the constriction (17) and at a distance (b) therefrom, and the metering means (4) comprises a cannula (42) projecting into the pressure chamber (1) sufficiently far that the opening (42a) of the cannula, the constriction (17) and the cell holder (25) are located essentially on a straight line parallel to or coinciding with the longitudinal axis (A) of the pressure chamber (1).

16. An apparatus according to claim 15, wherein the metering means (4) comprises an adjustable metering device (44 - 49) that feeds the cannula (42) and that is capable of allowing formation of uniform drops (T) of suspension of defined variable volume at the opening (42a) of the cannula (42).

17. An apparatus according to either claim 15 or claim 16, wherein the angle ($\alpha$) at which the cannula (42) opens into the pressure chamber (1) is variable.

18. An apparatus according to any one of claims 15 to 17, wherein the pressure chamber (1) is tapered towards the constriction (17) at its end facing the constriction.

19. An apparatus according to claim 17, wherein the pressure chamber (1) is arranged to be rotated, at least to a limited extent, about its longitudinal axis (A).

20. An apparatus according to any one of claims 15 to 19, which comprises an at least partially evacuable specimen chamber (2) equipped with a connection (28) for a source (5) of suction, into which chamber the constriction (17) opens and in which the cell holder (25) is located.

21. An apparatus according to claim 20, wherein the cell holder (25) is located inside the specimen chamber (2) in such a manner that it is displaceable at least transversely relative to the longitudinal direction (A) of the pressure chamber (1).

22. An apparatus according to claim 21, wherein the specimen chamber (2) comprises a removable part (23), and the cell holder (25), together with adjusting means (27) for the spatial displacement thereof, is located in the removable part (23) of the specimen chamber (2).

23. An apparatus according to any one of claims 15 to 22, wherein a non-return valve (43) is provided in the cannula (42) or in a supply line to the cannula.

24. An apparatus according to any one of claims 15 to 23, wherein the device for generating a pulse of pressure is a pistol-like device (31) operated by compressed air or compressed gas.

25. An apparatus according to any one of claims 15 to 24, wherein the constriction (17) is a diaphragm or a capillary.

26. An apparatus according to claim 25, wherein the free diameter of the diaphragm is about 20 - 300 $\mu$m.

27. An apparatus according to claim 25, wherein the internal diameter of the capillary (17) is about 10 - 500 $\mu$m and the length of the capillary (17) is about 1 - 20 mm.

28. An apparatus according to any one of claims 15 to 27, wherein the distance from the opening (42a) of the cannula (42) to the constriction (17) is about 5 - 20 mm.

29. A process according to claim 1, wherein said cells are present as a component of a more highly organised plant unit.

30. A process according to claim 29, wherein the unit is a piece of tissue with only a few cells.

31. A process according to claim 30, wherein the unit is a piece of meristematic tissue.

**32.** A process according to any one of claims 1 to 13, wherein the cells to be transformed are plant proembryos or plant embryos or embryogenic plant cell cultures.

**33.** A process for the preparation of a transgenic plant, wherein a plant cell prepared in accordance with the process according to any one of claims 1 to 13 or 29 to 32 is regenerated to a whole transgenic plant.

**34.** A process according to any one of claims 1 to 13, wherein the said cell is a prokaryotic cell.

**35.** A process according to any one of claims 1 to 13, wherein the said cell is a bacterial cell.

**Revendications**

**1.** Procédé de transformation génétique de cellules, notamment de cellules végétales, par bombardement des cellules par des microprojectiles entraînant avec eux de l'ADN (acide désoxynucléique), notamment des particules d'or ou de tungstène de grosseur micrométrique envoyées avec une impulsion ou une quantité de mouvement telle que les microprojectiles pénètrent dans les cellules à transformer ou tout au moins endommagent tellement ces cellules que l'ADN peut pénétrer dans les cellules, procédé dans lequel les microprojectiles sont mis en suspension dans une solution d'ADN et un volume bien défini de cette suspension est préparé, procédé caractérisé en ce que le volume bien défini de suspension est préparé à l'intérieur d'une chambre (1) de pression et en ce que les microprojectiles sont finement pulvérisés ou atomisés, à l'intérieur de la chambre de pression, par un coup de pression qui les fait sortir du volume bien défini déjà préparé de cette suspension, avec la solution, et les accélère pour les faire sortir de la chambre de pression en direction des cellules à transformer.

**2.** Procédé selon la revendication 1, caractérisé en ce que le nuage résultant de la pulvérisation fine ou de l'atomisation, nuage contenant les microprojectiles et l'ADN qui y adhère, est pressé pour le refouler à travers une restriction (17), dirigée vers les cellules à transformer et ayant la forme d'un diaphragme ou d'un capillaire, et ce nuage est ainsi accéléré et focalisé ou concentré vers les cellules.

**3.** Procédé selon la revendication 1 ou 2, caractérisé en ce que les cellules à transformer sont placées, au moins pendant leur bombardement par des microprojectiles, dans un espace (2) au moins partiellement mis sous dépression.

**4.** Procédé selon l'une des revendications 1 à 3, caractérisé en ce que chaque goutte de solution d'ADN (T) est préparée avec les microprojectiles qui sont en suspension, et cette goutte est ensuite atomisée par le coup de pression.

**5.** Procédé selon la revendication 4, caractérisé en ce que la goutte (T) est disposée sur une ligne droite (A) passant par la restriction (17) et par les cellules et en ce que la direction de dispersion principale du coup ou du tir de pression est choisie de manière à être constamment colinéaire avec cette ligne.

**6.** Procédé selon l'une des revendications 1 à 5, caractérisé en ce qu'on utilise comme microprojectiles des particules, notamment des particules d'or de grosseur essentiellement homogène se situant dans le domaine de 20 nm à 5 nm, avantageusement de 1,2 à 1,5 nm de diamètre.

**7.** Procédé selon la revendication 4, caractérisé en ce que le volume d'une goutte (T) est choisi dans le domaine ou intervalle compris entre 10 et 500 nl, avantageusement environ 50 à 150 nl.

**8.** Procédé selon les revendications 2 et 4, caractérisé en ce que la distance entre la goutte (T) et la restriction (17) est choisie de préférence dans un domaine ou intervalle d'environ 5 à 20 mm en étant assez grande pour qu'aucun fil fermé de liquide ne puisse pénétrer dans la restriction.

**9.** Procédé selon la revendication 4, caractérisé en ce que la goutte (T) est produite à l'extrémité de sortie (42a) d'une canule (42) inclinée par rapport à la verticale (V), l'angle ($\alpha$) de la canule par rapport à la verticale (V) étant choisi, en fonction de la grosseur des microprojectiles et de la viscosité de la solution d'ADN contenant les microprojectiles en suspension, de façon que la concentration des microprojectiles en suspension demeure essentiellement constante dans les gouttes successives.

10. Procédé selon la revendication 2, caractérisé en ce que la restriction (17) présente un diamètre de libre passage se situant entre 20 et 300 $\mu$m.

11. Procédé selon la revendication 2, caractérisé en ce qu'on utilise comme restriction un capillaire (17) ayant un diamètre intérieur de 10 $\mu$m à 500 $\mu$m et ayant une longueur de 1 à 20 mm.

12. Procédé selon l'une des revendications 1 à 11, caractérisé en ce que la poussée ou le tir de pression est choisi dans l'intervalle (de pression) de 2 à 10 000 bars, avantageusement dans l'intervalle de quelques dizaines de bars à quelques centaines de bars.

13. Procédé selon l'une des revendications 1 à 12, caractérisé en ce qu'on travaille avec une concentration en ADN de l'ordre de quelques dixièmes de microgramme par microlitre.

14. Appareil ou dispositif pour introduire des particules entraînant de l'ADN dans des cellules, notamment végétales, en particulier pour la transformation génétique de ces cellules, l'appareil comportant un support (25) pour les cellules, des milieux ou moyens (4) pour préparer une suspension d'un volume défini contenant des particules et de l'ADN et des moyens (3) pour accélérer les particules vers le porte-cellules, appareil caractérisé en ce qu'une chambre (1) de pression est prévue, en ce que les moyens de dosage (14) préparent le volume défini (T) à l'intérieur de la chambre (1) de pression et en ce que les moyens (3) pour accélérer les particules sont réalisés en vue d'atomiser ce volume (T) à l'intérieur de la chambre (1) de pression en donnant un fin nuage et pour accélérer ce nuage pour le faire sortir de la chambre (1) de pression et le diriger vers le porte-cellules (25).

15. Appareil selon la revendication 14, caractérisé en ce que la chambre (1) de pression s'étend essentiellement dans le sens de sa longueur ; en ce qu'à une extrémité de la chambre de pression est placé un dispositif (31) pour produire un coup ou tir de pression et en ce que l'autre extrémité débouche dans une restriction (17) ; en ce que le porte-cellules (25) est placé dans le prolongement de la chambre (1) de pression en étant aligné avec la restriction (17), à une distance (b) de cette restriction ; et en ce que les moyens de dosage (4) présentent une canule (42) pénétrant dans la chambre (1) de pression sur une longueur telle que le débouché (42a) de la canule, la restriction (17) et le porte-cellules (25) sont situés essentiellement sur une ligne droite parallèle à l'axe longitudinal (A) de la chambre (1) de pression coïncidant avec cet axe.

16. Appareil selon la revendication 15, caractérisé en ce que les moyens de dosage (4) comprennent un dispositif de dosage (44-49) réglable pour alimenter la canule (42), le dispositif étant capable de provoquer la présence, au débouché (42a) de la canule (42), d'une goutte de suspension demeurant la même et ayant un volume bien défini et réglable.

17. Appareil selon la revendication 15 ou 16, caractérisé en ce que l'angle ($\alpha$) selon lequel la canule (42) pénètre dans la chambre (20) de pression est réglable.

18. Appareil selon l'une des revendications 15 à 17, caractérisé en ce que la chambre (1) de pression a, vers la restriction (17), une forme qui s'effile à son extrémité située vers la restriction.

19. Appareil selon la revendication 17, caractérisé en ce que la chambre (1) de pression est disposée de façon à pouvoir tourner de manière au moins limitée autour de son axe longitudinal (A).

20. Appareil selon l'une des revendications 15 à 19, caractérisé en ce qu'il présente une chambre (2) à échantillon, au moins partiellement sous dépression, comportant un tube pour le raccordement (28) d'une source (5) d'aspiration, chambre dans laquelle débouche la restriction (17) et dans laquelle est disposé le porte-cellules (25).

21. Appareil selon la revendication 20, caractérisé en ce que le porte-cellules (25) est disposé à l'intérieur de la chambre (2) à échantillon en étant au moins partiellement déplaçable perpendiculairement à la direction longitudinale (A) de la chambre (1) de pression.

22. Appareil selon la revendication 21, caractérisé en ce que la chambre (2) à échantillon présente une partie (25) amovible et en ce que le porte-cellules (25) avec les moyens d'ajustement ou de réglage (27) pour le déplacement de l'endroit de disposition du porte-cellules sont placés dans cette partie (23) amovible de la chambre (2) à échantillon.

23. Appareil selon l'une des revendications 15 à 22, caractérisé en ce que une soupape de non-retour (43) est prévue dans la canule (42) ou dans un conduit amenant à cette canule.

24. Appareil selon l'une des revendications 15 à 23, caractérisé en ce que le dispositif destiné à produire un coup ou tir de pression est un appareil (31) du genre pistolet pouvant fonctionner à l'aide d'air comprimé ou de gaz comprimé.

25. Appareil selon l'une des revendications 15 à 24, caractérisé en ce que la restriction (17) est un diaphragme ou un capillaire.

26. Appareil selon la revendication 25, caractérisé en ce que le diamètre libre du diaphragme vaut environ 20 à 300 $\mu$m.

27. Appareil selon la revendication 25, caractérisé en ce que le diamètre intérieur du capillaire (17) a environ 10 à 500 $\mu$m et la longueur du capillaire (17) est d'environ 1 à 20 ml.

28. Appareil selon l'une des revendications 15 à 27, caractérisé en ce que la distance entre le débouché (42a) de la canule (42) et la restriction (17) est d'environ 5 à 20 mm.

29. Procédé selon la revendication 1, caractérisé en ce que lesdites cellules font partie d'une unité ou d'un organisme végétal organisé supérieur.

30. Procédé selon la revendication 29, caractérisé en ce qu'il s'agit d'un groupement de tissus comportant un petit nombre de cellules.

31. Procédé selon la revendication 30, caractérisé en ce qu'il s'agit d'un groupement ou ensemble de tissus méristématiques.

32. Procédé selon l'une des revendications 1 à 13, caractérisé en ce qu'il s'agit, pour les cellules à transformer, de pro-embryons végétaux ou d'embryons végétaux ou de cultures de cellules végétales embryogènes.

33. Procédé de préparation d'une plante transgénique, caractérisé en ce qu'on soumet une cellule végétale, produite selon le procédé selon l'une des revendications 1 à 13 ou 29 à 32, à une régénération donnant une plante transgénique complète.

34. Procédé selon l'une des revendications 1 à 13, caractérisé en ce qu'il s'agit pour lesdites cellules d'une cellule procaryote.

35. Procédé selon l'une des revendications 1 à 13, caractérisé en ce qu'il s'agit pour lesdites cellules d'une cellule bactérienne.

**Fig. 1**

EP 0 434 616 B1

Fig. 2

Fig. 3

EP 0 434 616 B1